# EUROPEAN PATENT APPLICATION

(11) **EP 4 014 980 A1**
(43) Date of publication of application: **22.06.2022**
(21) Application number: 19940811.3
(22) Date of filing: 08.08.2019
(51) Int. Cl.: A61K 33/20, A01N 59/08, A01P 1/00, A61P 31/02

(54) **HALAL DISINFECTANT LIQUID**

(71) Applicant: Sankei Co., Ltd., Chuo-ku Osaka-shi Osaka 540-0001 (JP)
(72) Inventor: GODA, Hisataka, Osaka-shi, Osaka 541-0048 (JP)
(74) Representative: Müller-Boré & Partner Patentanwälte PartG mbB
(86) International application number: PCT/JP2019/031501
(87) International publication number: WO 2021/024485

(57) **Abstract**

The present disclosure provides a disinfectant liquid for disinfecting an animal. The present disclosure provides a disinfectant liquid that contains chlorous acid water, and that is used for disinfection of an animal by bringing the disinfectant liquid into contact with the animal. The contact is achieved by a rubbing method, a scrubbing method, or a basin method. The animal is a living animal. The animal includes an animal surface. The animal surface is skin. The animal surface is a finger. The animal includes a human body. The disinfection is performed in the preparation of food. The disinfectant liquid contains no animal-derived components, alcoholic components, or fragrances.

## Description

### [Technical Field]

The present disclosure relates to a liquid for microbe-removal comprising a chlorous acid aqueous solution for removing microbes of an animal by directly contacting the liquid for microbe-removal with the animal.

### [Background Art]

Disinfection of the body, especially the hands and fingers, is demanded from all people working for a food processing company or a distributor of food such as processed food, people who prepare or cook food, people utilizing restaurant facilities including diners and food preparation facilities including a kitchen, all people who access such places, ingredient suppliers, waiters, servers, people who come in direct contact with food, people who come in indirect contact with food, etc. It is necessary to routinely wash hands to prevent food poisoning incidents and infections due to food-poisoning-causing microorganisms and pathogenic microorganism including viruses such as norovirus.

A liquid for microbe-removal that is halal compliant has not been developed.

### [Summary of Invention]

### [Solution to Problem]

As a result of diligent study, the inventors completed by the present disclosure by discovering that a liquid for microbe-removal comprising a chlorous acid aqueous solution is capable of safe microbe removal while directly contacting an animal.

For example, the present invention provides the following items.

### (Item 1)

A liquid for microbe-removal comprising a chlorous acid aqueous solution for removing microbes of an animal by directly contacting the liquid for microbe-removal with the animal.

### (Item 2)

The liquid for microbe-removal of item 1, wherein the contact is achieved by a rubbing method, a scrubbing method, or a basin method.

### (Item 3)

The liquid for microbe-removal of item 2, wherein the phosphate buffer comprises two or more types of phosphates.

### (Item 4)

The liquid for microbe-removal of item 2 or 3, wherein the phosphate buffer comprises two or more of potassium dihydrogen phosphate, dipotassium hydrogen phosphate, and sodium dihydrogen phosphate.

### (Item 5)

The liquid for microbe-removal of item 4, wherein potassium dihydrogen phosphate is 0.68% to 13.61%, and sodium dihydrogen phosphate is 0.60% to 12.00%.

### (Item 6)

The liquid for microbe-removal of item 4, wherein potassium dihydrogen phosphate is 1%, and sodium dihydrogen phosphate is 1%.

### (Item 7)

The liquid for microbe-removal of item 4, wherein potassium dihydrogen phosphate is 0.68% to 13.61%, and dipotassium hydrogen phosphate is 0.87% to 17.42%.

### (Item 8)

The liquid for microbe-removal of item 4, wherein potassium dihydrogen phosphate is 1%, and dipotassium hydrogen phosphate is 1%.

### (Item 9)

The liquid for microbe-removal of any one of items 1 to 8, wherein the animal is a live animal.

### (Item 10)

The liquid for microbe-removal of any one of items 1 to 9, wherein the animal comprises a surface of the animal.

### (Item 11)

The liquid for microbe-removal of item 10, wherein the surface of the animal is skin.

### (Item 12)

The liquid for microbe-removal of item 11, wherein the surface of the animal is a hand or a finger.

### (Item 13)

The liquid for microbe-removal of any one of items 1 to 12, wherein the animal comprises a human body.

### (Item 14)

The liquid for microbe-removal of any one of items 1 to 13, wherein the removal of microbes is performed during preparation of food.

### (Item 15)

The liquid for microbe-removal of any one of items 1 to 14, wherein chlorous acid is used at 10 ppm to 60,000 ppm.

### (Item 16)

The liquid for microbe-removal of any one of items 1 to 15, wherein chlorous acid is used at 200 ppm to 8,000 ppm.

### (Item 17)

The liquid for microbe-removal of any one of items 1 to 16, wherein a free chlorine concentration of a chlorous acid aqueous solution is 0.25 mg/L to 1,500 mg/L.

### (Item 18)

The liquid for microbe-removal of any one of items 1 to 17, wherein the removal of microbes targets at least one selected from a virus, a microbe, and a eukaryote.

### (Item 19)

The liquid for microbe-removal of any one of items 1 to 18, wherein the liquid for microbe-removal does not contain an animal derived component, an alcohol component, or a flavor.

### (Item 20)

A halal-compliant hand and finger liquid for microbe-removal, comprising 1.00% chlorous acid aqueous solution, 1.00% sodium dihydrogen phosphate, 1.00% potassium dihydrogen phosphate, and 97.00% ion exchange water, wherein chlorous acid is 400 ppm, and a free chlorine concentration is 10 mg/L.

### (Item 21)

A halal-compliant hand and finger liquid for microbe-removal, comprising 20.00% chlorous acid aqueous solution, 1.00% potassium dihydrogen phosphate, 1.00% dipotassium hydrogen phosphate, and 78.00% ion exchange water, wherein chlorous acid is 8,000 ppm, and a free chlorine concentration is 200 mg/L.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

### [Advantageous Effects of Invention]

Sodium hypochlorite that is used in the field of disinfecting agents, when directly sprayed onto the site to be disinfected or when the site is immersed directly therein, results in rough hands, etc. due to strong irritation to the skin, so that direct use is not preferred. The dosage and administration determines that sodium hypochlorite is used at an effective chlorine concentration of 0.01 to 0.05% (100 ppm to 500 ppm) for approval of drugs. This concentration is higher than 0.001 %, which is the minimum concentration resulting in cytotoxicity (10 ppm; Japanese Journal of Infectious Diseases Vol. 71 (2018), No. 5 pp. 333-337), so that use thereof as a pharmaceutical product is restricted to very limited situations. Meanwhile, it was found that the chlorous acid aqueous solution of the present disclosure does not have such a limitation. Use of povidone iodine formulations used in the field of disinfecting agents is not preferable in cases of iodine hypersensitivity or in cases of problems with the thyroid function, and such a formulation irritates the skin and has a color (brown). Thus, the formulation is used only for disinfection of skin before surgical incisions (swabbing method: absorbent cotton is immersed therein and wiped). Meanwhile, it was found that the chlorous acid aqueous solution of the present disclosure does not have such a limitation. While alcohols used in the field of disinfecting agents also cause irritation to the skin, it was found that the chlorous acid aqueous solution of the present disclosure causes hardly any irritation. Since alcohols are hazardous materials (class IV alcohol) in addition to being irritable, there is a restriction for the location of storage/installation and in some cases the quantity. The chlorous acid aqueous solution of the present disclosure has an effect of having a disinfecting effect on noroviruses and alcohol-resistant bacteria. Use of alcohol can be considered haram, so that no use of alcohol is demanded. Meanwhile, such a restriction does not apply to the chlorous acid aqueous solution of the present disclosure. Benzalkonium chlorides used in the field of disinfecting agents are low-level disinfecting agents, which are effective on general bacteria, yeast-like fungi, etc., and have a narrow antibacterial spectrum. Meanwhile, it was found that the chlorous acid aqueous solution of the present disclosure has a disinfecting effect on a broad antibacterial spectrum.

The present application provides a liquid for microbe-removal capable of removing microbes of an animal by directly contacting the animal.

A liquid for microbe-removal comprising a chlorous acid aqueous solution can be contacted directly with an animal. Thus, animals can be disinfected thoroughly by using a disinfection method with a high microbe-removing effect such as a rubbing method, scrubbing method, or basin method. All four disinfecting methods (rubbing method, scrubbing method, swabbing method, and basin method) can be used for the chlorous acid aqueous solution and chlorous acid aqueous solution formulation of the present disclosure. Since chlorous acid aqueous solutions and chlorous acid aqueous solution formulations fall under high-level disinfecting agents that can also sterilize and disinfect spores and can be used in each disinfecting method, chlorous acid aqueous solutions and chlorous acid aqueous solution formulations have an advantage of allowing combined management with a single agent. When "directly sprayed onto the hand and fingers, or the hand and fingers is immersed directly therein", the chlorous acid aqueous solution and chlorous acid aqueous solution formulation is directly used on the target of disinfection. Thus, the chlorous acid aqueous solution and chlorous acid aqueous solution formulation can be used at a lower concentration. "Directly sprayed onto the hand and fingers, or the hand and fingers is immersed directly therein" is a method which is simpler and has a higher disinfecting effect than a swabbing method using a wet sheet. Thus, a method for "directly sprayed onto the hand and fingers, or the hand and fingers is immersed directly therein" is better than a swabbing method in some aspects.

Since a liquid for microbe-removal comprising a chlorous acid aqueous solution can be an alcohol free, animal free, and flavor free microbe-removing agent, the solution can also be used in the manufacture of halal food.

### [Brief Description of Drawings]

[Figure 1] Figure 1 shows skin of a test substance treatment group of Example 2.
[Figure 2] Figure 2 shows skin of a comparative control substance treatment group of Example 2.
[Figure 3] Figure 3 shows the reaction of an eye in Example 3.
[Figure 4] Figure 4 shows a site treated with a test substance in Example 4.
[Figure 5] Figure 5 shows a site treated with a negative control group in Example 4.
[Figure 6] Figure 6 shows a site treated with a negative control group in Example 4.
[Figure 7] Figure 7 shows a site treated with a positive control group in Example 4.

### [Description of Embodiments]

The present disclosure is described in more detail hereinafter. Throughout the entire specification, a singular expression should be understood as encompassing the concept thereof in the plural form, unless specifically noted otherwise. Thus, singular articles (e.g., "a", "an", "the", and the like in case of English) should also be understood as encompassing the concept thereof in the plural form, unless specifically noted otherwise. The terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The abbreviations used herein have the conventional meaning within the scope of the art unless specifically noted otherwise.

The term "about" for a value or parameter herein includes variations about the value or parameter itself. Unless specifically noted otherwise, "about X" for example includes "X" itself as well as values with an acceptable error of ± 10% therefrom.

As used herein, "chlorous acid aqueous solution" refers to an aqueous solution comprising chlorous acid (HClO₂) used as a germicide, which creates a stable chemically equilibrium state and delays a reaction to decompose chlorous acid into chlorine dioxide so that chlorous acid (HClO₂) can be stably maintained over a long period of time. The presence of a chlorous acid aqueous solution can be confirmed when an absorbent section comprising an acidic chlorite ion (H⁺+ClO₂⁻) representing a peak near 260 nm and an absorbent section comprising chlorine dioxide (ClO₂) representing a peak near 350 nm can be simultaneously observed between wavelengths of 240 to 420 nm in the UV spectrum, i.e., when a double peak is exhibited, when a sample of chlorous acid aqueous solution is measured with a spectrophotometer. In such a case, it is understood that a cyclic reaction involving the main constituent chlorous acid (HClO₂), and chlorine dioxide (ClO₂) and acidic chlorite ion (H⁺+ClO₂⁻) is simultaneously in progress.

### [Chemical Formula 1]

Cyclic reaction among chlorous acid, chlorine dioxide, and acidic chlorite ion

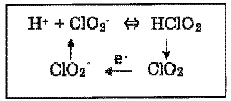

Chlorous acid aqueous solution can be prepared by the methods disclosed in International Publication No. WO 2008/026607, WO 2014/188310, WO 2014/188311, WO 2014/188312, WO 2015/093062, and WO 2017/170904.

"Chlorous acid aqueous solution" is a germicide that was designated as a food additive on February 1, 2013 and has chlorous acid (HClO₂) as the primary active ingredient. The primary active ingredient chlorous acid (HClO₂) of such a "chlorous acid aqueous solution" is a semi-stable chemical substance, which is approved by the USDA and FDA as a food additive: processing aid as an especially safe substance.

A cyclic reaction that maintains a chemical equilibrium relationship of chlorous acid (HClO₂) can be utilized for stabilization for a long period of time in a solution while maintaining a non-dissociated state.

Moreover, "chlorous acid aqueous solution" can exert a potent germicidal effect even in the presence of an organic matter. Chlorous acid aqueous solution was highly praised as "only chlorous acid aqueous solution was capable of inactivation to the detection limit or below under all load conditions" in "Heisei 27 Nendo Norouirusu no Fukatsuka Joken ni Kansuru Chosa [2015 Investigative Report on Norovirus Inactivation Conditions]" at the National Institute of Health Sciences (NIHS). With the revisions of the Ordinance for Enforcement of the Food Sanitation Act, listing of chlorous acid aqueous solution is ongoing in order of incidents of large scale food poisoning such as "Tairyo Chori Shisetsu Chori Manyuaru [Manual for Food Preparation at Large-scale Food Preparation Facilities]" and "Tsukemono no Eisei Kihan [Code of Hygienic Practice for Pickles]".

In addition, application for a pharmaceutical product as a class II germicidal disinfectant is pending with scheduled sale in 2020. With this background, chlorous acid aqueous solution is added to guidelines such as "Hoikusho ni okeru Kansen Taisaku Manyuaru [Infection Control Measure Guideline at Nursery Center]" under administration of the Ministry of Health, Labour and Welfare including "Norouirusu ni Kansuru Q&A [Q&A regarding noroviruses]", relevant manuals such as "Koreisha Kaigo Shien Shisetsu ni okeru Kansen Taisaku Manyuaru [Manual for Controlling Infection at Elderly Care Support Facility]", various sanitation codes such as "Bento/Sozai no Eisei Kihan [Code of Sanitation for Bento/Dishes]", etc., which are also being revised as needed. Chlorous acid aqueous solution is a substance that is supplied to wide ranging markets in food sanitation and environmental sanitation markets in Japan.

"Chlorous acid aqueous solution" with chlorous acid as the primary active ingredient possesses a germicidal power that is equivalent to or greater than that of "sodium hypochlorite water" and "hypochlorous acid water". "Chlorous acid aqueous solution" with a characteristic of having no instantaneous germicidal effect (immediate effect), but having a gradual reactivity while having a precise germicidal power, as well as sustained stable germicidal power, can exert a germicidal effect that is precise and accurate, albeit slowly, under a contaminated environment with many organic matters, which had been considered as the most challenging for conventional chlorous oxide based agents (germicidal power against microorganisms that are latent in contamination).

For this reason, chlorous acid aqueous solution can exert an inactivation effect on resistant bacteria that had been difficult to sterilize in the past (heat-resistant bacteria with increased resistance by forming spores, drug resistant bacteria on which antibiotics are no longer effective, etc.), fungi such as mold and yeast, and viruses (including non-enveloped viruses). "Chlorous acid aqueous solution" does not require preparation upon use or a dedicated generator therefor. Chlorous acid aqueous solution is safe and can be used by anyone at anywhere.

In addition, the effect of "chlorous acid aqueous solution" in the presence of organic matter is listed in "Heisei 27 Nendo Norouirusu no Fukatsuka Joken ni Kansuru Chosa Hokokusho [2015 Investigative Report on Norovirus Inactivation Conditions] (National Institute of Health Sciences, Division of Biomedical Food Research)" on the website of Ministry of Health, Labour and Welfare.

A germicidal effect of chlorous acid aqueous solution on various microorganisms and viruses based on free chlorine concentration is summarized as follows.

### [Chemical formula 2]

| Bacterial species | Free chlorine (mg/L) with which disinfection effect is achieved (3 Log reduction within 10 minutes) |
|---|---|
| Escherichia coli NBRC3972 | 1 mg/L [0.5% BSA: 13 mg/L] |
| Escherichia coli O157: H7) | 1 mg/L [0.5% BSA: 17 mg/L] |
| Staphylococcus aureus NBRC12732 | 1 mg/L [0.5% BSA: 50 mg/L] |
| Methicillinresistant Staphylococcus Aureus (MRSA) | < 50 mg/L [0.5% BSA: 50 mg/L] |
| Pseudomonas aeruginosa AOAC110401 | < 50 mg/L [0.5% BSA: 100 mg/L] |
| Mycobacterium intracellulare | < 50 mg/L [0.5% BSA: 100 mg/L] |
| Bacillus cereus NBRC15305 (spore) | 50 mg/L |
| Clostridium difficile (Spore) | < 50 mg/L [0.5% polypeptone: 100 mg/L] |
| Candida albicans NBRC1594 | 5 mg/L |
| Rhodotorula mucilaginosa NBRC0909 | 5 mg/L |
| Aspergillus flavus NBRC33021 | 5 mg/L |
| Penicillium thomii NBRC31394 | 5 mg/L |
| feline calicivirus F9 (FCV) | 5 mg/L |
| | [0.5% BSA: 50 mg/L, 0.5% polypeptone: 100 mg/L] |

As used herein, "microbe-removing (action)" refers to removal of pathogenic, harmful, or infectious microorganisms such as filamentous fungi, bacteria, or viruses. "Microbe-removing (action)" is used as a broad concept encompassing not only microbe-removing (action), but also antimicrobial (action), germicidal (action), and disinfecting (action), unless specifically noted otherwise. Thus, substances having antimicrobial action, germicidal action, microbe-removing action, or disinfecting action are collectively referred herein as a "microbe-removing agent", which is understood as an agent also having action corresponding to antimicrobial action, germicidal action, microbe-removing action, and disinfecting action in general use herein.

Examples of microorganisms removed herein include microorganisms that can cause food poisoning, pathogenic microorganisms, microorganisms that can induce an infection, drug resistant bacteria (microbes), and the like. Examples thereof include fecal coliforms, pathogenic E. coli, Staphylococcus aureus, Salmonella bacteria, Vibrio parahaemolyticus, Campylobacter, Clostridium perfringens, Bacillus cereus, and the like. The relationship of microorganisms that can cause food poisoning, pathogenic microorganisms, microorganism that can induce an infection, and drug resistant bacteria (microbes) is the following.

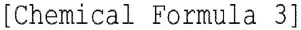

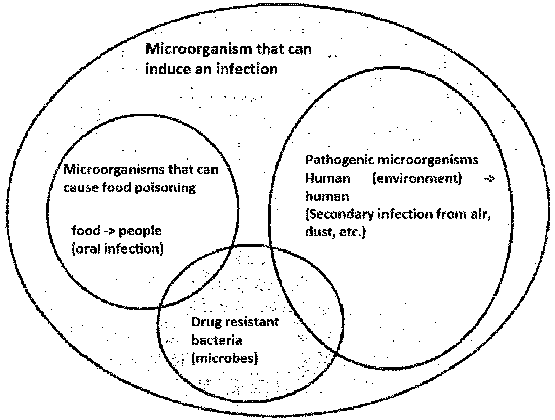

As used herein, "infection" refers to an infection from a human, animal, environment, or the like, from which the amount of pathogen within the body reaches a certain amount, resulting in manifestation of a symptom in a host such as fever, diarrhea, or coughing. An infection can also develop from a small amount of microorganisms. For viruses, an infected host can be a carrier by coexisting with a virus over an extended period (persistent infection), or an infection can be an infection that does not result in onset of a disease (latent infection). "Infect" refers to a pathogen entering the body of a host (human or animal) and growing and proliferating in the body. "Pathogen" refers to microorganism (microbes, viruses, fungi, rickettsiae, protozoa, and parasites) inducing a disease from infection, and pathogens excluding parasites are referred to as "pathogenic microorganisms". Examples thereof include class I infections (Ebola hemorrhagic fever, Crimean-Congo hemorrhagic fever, smallpox, South American hemorrhagic fever, pest, Marburg disease, Lassa fever, etc.), class II infections (acute poliomyelitis (polio), tuberculosis, diphtheria, SARS, etc.), class III infections (cholera, shigellosis, enterohemorrhagic Escherichia coli infection, abdominal typhus, paratyphoid fever, etc.), class IV infections (hepatitis A/E, malaria, yellow fever, Q fever, rabies, botulism, malaria, etc.), and class V infections (influenza, viral hepatitis, cryptosporidium, AIDS, genital chlamydial infection, syphilis, measles, MRSA, norovirus, etc.).

As used herein, "food poisoning" refers to oral infection through ingestion of food (oral intake), resulting in a symptom (case). Food poisoning is a type of infection, which is a food mediated infection. An infection is induced by a microorganism that is present in an animal (livestock) or nature. Intake of meat or the like results in many food poisoning incidents every year. Microbial food poisoning includes infectious food poisoning such as invasive type (Salmonella, etc.) and toxigenic type (Vibrio parahaemolyticus, pathogenic E. coli, etc.), and toxigenic food poisoning from Staphylococcus aureus, botulism, etc. Examples of viral food poisoning include food poisoning due to a norovirus, hepatitis A virus, hepatitis E virus, or the like. Examples of chemical food poisoning include food poisoning due to a hazardous food additive (arsenic, residual pesticide, etc.), contaminant, etc. Examples of natural food poisoning include animal derived food poisoning due to a natural toxin such as puffer fish toxin and shellfish toxin, and vegetable derived food poisoning due to natural toxin such as poison mushrooms and poisonous plants. Examples of food poisoning also include allergic food poisoning.

As used herein, "pathogenic microorganism" refers to microorganisms infecting through a human or environment, excluding food poisoning (oral infection). Infected patients can disperse microorganisms through diarrhea, vomit, cough, etc. to cause infections through secondary infection.

As used herein, "drug resistance" refers to a phenomenon of having resistance to a drug, such as antibiotics, having some type of an effect on a microbe itself and thereby such drugs becoming ineffective or less effective thereon. This results from acquiring resistance to a drug (antibiotic) within the body upon treatment through administration of the antibiotic when an animal (human) has an infection. All bacterial species may have developed resistance to some antibiotics. Examples of antibiotics include bacteriostatic agents (suppress bacterial growth) and germicides (kill bacteria).

As used herein, a "drug-resistant bacterium" refers to a bacterium that has acquired drug-resistance. Such a drug-resistant bacterium includes, but is not limited to, methicillin-resistant Staphylococcus aureus (MRSA), multidrug-resistant Pseudomonas aeruginosa (MDRP), vancomycin-resistant Enterococcus (VRE), Clostridium difficile (CD: sporulation, toxicogenic), vancomycin-resistant Staphylococcus aureus, multidrug-resistant Staphylococcus aureus, Pseudomonas aeruginosa, penicillin-resistant Streptococcus pneumoniae, multidrug-resistant Acinetobacter, Canbapenem antibacterial agent-resistant bacteria, multidrug-resistant Serratia, and the like. Although not wishing to be bound by any theory, a drug-resistant gene generally has acquired a gene imparting drug-resistance. The present invention is considered to have a microbe-killing effect by destroying such a gene or gene product. Thus, the present invention has demonstrated an effect on specific multidrug resistant bacteria, which are bacteria that can resist multiple drugs. Those skilled in the art understand that the present invention is extrapolated to generally have an effect on simpler drug-resistant bacteria.

As used herein, "multidrug resistant bacteria" refers to bacteria that have acquired resistance to a plurality of drugs (especially antibiotics).

As used herein, "liquid for microbe-removal" refers to a liquid microbe-removing agent.

As used herein, "directly contacting the liquid for microbe-removal with the animal" refers to contacting a liquid for microbe-removal with an animal by spraying or immersing a portion of an animal to be disinfected in a liquid for microbe-removal. As used herein, this does not include wiping with a liquid for microbe-removal impregnated in absorbent cotton, gauge, treatment sheet, or the like (swabbing method).

As used herein, "rubbing method" (also known as waterless method) is a method of rubbing in a liquid for microbe-removal into a site to be disinfected (e.g., hand and fingers) until it is dry. This is performed, when pump spraying is used, by spraying a liquid for microbe-removal onto the palm of a hand and rubbing in the solution well with both hands, and then removing moisture thoroughly with a nonwoven fabric, hand dryer, etc. to dry the hands. This is the most effective and simple method.

As used herein, "scrubbing method" is a method of allowing a liquid for microbe-removal combined with a detergent to foam up and washing the solution off with water. After washing the hands, moisture is completely wiped away using paper towel or the like. A brush is used in some cases. Cleaning and disinfection can be simultaneously be achieved.

As used herein, "basin method" is a method of washing by placing a liquid for microbe-removal of a certain concentration in a basin (e.g., wash bowl, etc.) and immersing a site to be disinfected therein. This can be performed by immersing a clean nonwoven fabric or dish rag in a liquid for microbe-removal, firmly immersing a site to be disinfected, swabbing the site in the solution using the immersed nonwoven fabric or dish rag, and removing moisture thoroughly with a nonwoven fabric, hand dryer, etc. to dry the site to be disinfected.

As used herein, "swabbing method" is a method of sufficiently allowing a liquid for microbe-removal to be soaked up by absorbent cotton, gauge, nonwoven fabric, or the like and wiping off the site to be disinfected. A swabbing method has the weakest effect compared to the other methods described above. A swabbing method does not fall under directly contacting a liquid for microbe-removal with an animal herein.

As used herein, "surface of an animal" refers to the surface of the body of an animal such as the skin, hair, nail, wing, scale, horn, or tooth of the animal. The surface also encompasses sites that cannot be readily contacted from the outside such as inside of the mouth.

As used herein, "free chlorine", "free chlorine concentration", or "free residual chlorine concentration" is a value that is measured according to Appendix 3 in "Testing method for free residual chlorine and bound chlorine specified by the Minister of Health, Labour and Welfare based on the stipulation of Article 17(2) of the regulation of the Water Supply Act" (hereinafter, colorimetry (DPD indicator)), and is a value obtained by oxidation of a DPD indicator.

As used herein, "concentration of chlorous acid aqueous solution" indicates a value measured by the (Method of quantifying chlorous acid aqueous solution) described below.

As used herein, "high-level disinfecting agent (liquid for microbe-removal)" is a classification of a chemical disinfecting method by classification according to Spaulding (Rutala WA: APIC Guideline for selection and use of disinfectants. 1996. Am J Infect Control 1996; 24: 313-342). This refers to a disinfecting agent that kills all microorganisms, except when many spores are present. Examples thereof include Glutaral formulation, phtaral formulation, peracetic acid formulation, etc.

As used herein, "moderate-level disinfecting agent (microbe-removing agent)" is a classification of a chemical disinfecting method by classification according to Spaulding (Rutala WA: APIC Guideline for selection and use of disinfectants. 1996. Am J Infect Control 1996; 24: 313-342). This refers to a disinfecting agent that kills Mycobacterium tuberculosis, vegetative bacteria, most fungi, and most viruses, but does not necessarily kill spores. Examples thereof include sodium hypochlorite, povidone-iodine formulations, alcohols, etc.

As used herein, "low-level disinfecting agent (microbe-removing agent)" is a classification of a chemical disinfecting method by classification according to Spaulding (Rutala WA: APIC Guideline for selection and use of disinfectants. 1996. Am J Infect Control 1996; 24: 313-342). This refers to a disinfecting agent that kills most vegetative bacteria, a certain type of fungi, and certain type of viruses. Examples thereof include chlorhexidine formulations, quaternary ammonium salts, amphoteric surfactants, etc.

As used herein, "halal-compliant" refers to being free of anything that is "haram (forbidden)" under the teaching of Islam. Examples of "haram" include alcohol, animal components, etc., which are set forth in the Halal food guideline (Department of Halal Certification EU). As used herein, "halal-compliant" refers to the manufacture from raw materials on a production line without including "haram" such as alcohol, animal components, flavors, etc.

### (Preferred embodiments)

One aspect of the present disclosure provides a liquid for microbe-removal comprising a chlorous acid aqueous solution for removing microbes from an animal by directly contacting the liquid for microbe-removal with the animal. Since the liquid for microbe-removal of the present disclosure directly contacts an animal, microorganisms such as filamentous fungi, microbes, and viruses can be reliably removed, with a low possibility of remaining. The liquid for microbe-removal of the present disclosure has low irritability even when directly contacting with an animal, so that the solution does not cause any damage.

The contact is achieved herein by a rubbing method, scrubbing method, or basin method. A higher microbe-removing effect is achieved compared to a swabbing method. Meanwhile, such methods have safety concerns due to a higher level of direct contact with skin compared to a swabbing method. Meanwhile, it was unexpectedly found that the chlorous acid aqueous solution of the present disclosure surprisingly does not damage the skin of a hand or fingers even when a solution is directly contacted with an animal by a rubbing method, scrubbing, method, basis method, etc.

A liquid for microbe-removal comprising phosphate buffer combined with 0.5% to 20% of 40,000 ppm undiluted chlorous acid aqueous solution has 200 ppm to 8000 ppm of chlorous acid. If 1% of 40,000 ppm undiluted chlorous acid aqueous solution is combined, chlorous acid would be 400 ppm. If 0.5% to 20% of 4% to 6% (40,000 ppm to 60,000 ppm) undiluted chlorous acid aqueous solution is combined, chlorous acid would be 200 ppm to 12000 ppm.

In the present disclosure, the phosphate buffer comprises two or more types of phosphates.

In the present disclosure, the phosphate buffer comprises two or more of potassium dihydrogen phosphate, dipotassium hydrogen phosphate, and sodium dihydrogen phosphate.

In the present disclosure, potassium dihydrogen phosphate is 0.68% to 13.61%, and sodium dihydrogen phosphate is 0.60% to 12.00%. Potassium dihydrogen phosphate can be 0.7%, 0.8%, 0.9%, 1.0%, 2.0%, 3.0%, 4.0%, 5.0%, 6.0%, 7.0%, 8.0%, 9.0%, 10%, 11%, 12%, or 13%, or any value therebetween. Sodium dihydrogen phosphate can be 0.60%, 0.7%, 0.8%, 0.9%, 1.0%, 2.0%, 3.0%, 4.0%, 5.0%, 6.0%, 7.0%, 8.0%, 9.0%, 10%, 11%, or 12%, or any value therebetween.

In the present disclosure, potassium dihydrogen phosphate is 1%, and sodium dihydrogen phosphate is 1%.

In the present disclosure, potassium dihydrogen phosphate is 0.68% to 13.61%, and dipotassium hydrogen phosphate is 0.87% to 17.42%. Potassium dihydrogen phosphate can be 0.7%, 0.8%, 0.9%, 1.0%, 2.0%, 3.0%, 4.0%, 5.0%, 6.0%, 7.0%, 8.0%, 9.0%, 10%, 11%, 12%, or 13%, or any value therebetween. Dipotassium hydrogen phosphate can be 0.9%, 1.0%, 2.0%, 3.0%, 4.0%, 5.0%, 6.0%, 7.0%, 8.0%, 9.0%, 10%, 11%, 12%, 13%, 14%, 15%, 16%, or 17%, or any value therebetween.

In the present disclosure, potassium dihydrogen phosphate is 1%, and dipotassium hydrogen phosphate is 1%.

In the present disclosure, the animal is a live animal.

In the present disclosure, the animal comprises a surface of the animal.

In the present disclosure, the surface of the animal is skin.

In the present disclosure, the surface of the animal is a hand or a finger.

In the present disclosure, the animal comprises a human body.

In the present disclosure, the removal of microbes is performed during preparation of food. Food preparation includes preparation at both a food processing plant and a food preparation facility (kitchen). Food preparation involves all people working for a food processing company or a distributor of food such as processed food, people who prepare or cook food, people utilizing restaurant facilities including diners and food preparation facilities including a kitchen, all people who access such places, ingredient suppliers, waiters, servers, people who come in direct contact with food, people who come in indirect contact with food, etc.

In the present disclosure, chlorous acid (as HClO₂ = 68.46) can be used at 10 ppm to 60,000 ppm (0.001% to 6%). Chlorous acid can be used at 200 ppm to 8,000 ppm. Chlorous acid can be used at 400 ppm to 8,000 ppm. Chlorous acid can be used at 200 ppm to 12,000 ppm. Chlorous acid can be used at about 10 ppm, about 15 ppm, about 20 ppm, about 25 ppm, about 30 ppm, about 40 ppm, about 50 ppm, about 60 ppm, about 70 ppm, about 80 ppm, about 90 ppm, about 100 ppm, about 150 ppm, about 200 ppm, about 250 ppm, about 300 ppm, about 350 ppm, about 400 ppm, about 450 ppm, about 500 ppm, about 550 ppm, about 600 ppm, about 650 ppm, about 700 ppm, about 750 ppm, about 800 ppm, about 850 ppm, about 900 ppm, about 950 ppm, about 1,000 ppm, about 1,500 ppm, about 2,000 ppm, about 2,500 ppm, about 3,000 ppm, about 3,500 ppm, about 4,000 ppm, about 4,500 ppm, about 5,000 ppm, about 5,500 ppm, about 6,000 ppm, about 6,500 ppm, about 7,000 ppm, about 7,500 ppm, about 8,000 ppm, about 8,500 ppm, about 9,000 ppm, about 9,500 ppm, about 10,000 ppm, about 11,000 ppm, about 12,000 ppm, about 13,000 ppm, about 14,000 ppm, about 15,000 ppm, about 20,000 ppm, about 25,000 ppm, about 30,000 ppm, about 35,000 ppm, about 40,000 ppm, about 45,000 ppm, about 50,000 ppm, about 55,000 ppm, about 60,000 ppm, or the like. The concentration of chlorous acid can be any value within a range thereof.

In the present disclosure, a free chlorine concentration (as Cl = 35.45) of a chlorous acid aqueous solution can be 0.25 mg/L to 1,500 mg/L. A free chlorine concentration of a chlorous acid aqueous solution can be used at about 0.25 mg/L, about 0.3 mg/L, about 0.35 mg/L, about 0.4 mg/L, about 0.45 mg/L, about 0.5 mg/L, about 0.55 mg/L, about 0.6 mg/L, about 0.65 mg/L, about 0.75 mg/L, about 0.8 mg/L, about 0.85 mg/L, about 0.95 mg/L, about 1 mg/L, about 2 mg/L, about 3 mg/L, about 4 mg/L, about 5 mg/L, about 6 mg/L, 7 mg/L, about 8 mg/L, about 9 mg/L, about 10 mg/L, about 11 mg/L, about 12 mg/L, about 13 mg/L, about 14 mg/L, about 15 mg/L, about 16 mg/L, about 17 mg/L, about 18 mg/L, about 19 mg/L, about 20 mg/L, about 21 mg/L, about 22 mg/L, about 23 mg/L, about 24 mg/L, about 25 mg/L, about 26 mg/L, about 27 mg/L, about 28 mg/L, about 29 mg/L, about 30 mg/L, about 35 mg/L, about 40 mg/L, about 45 mg/L, about 50 mg/L, about 55 mg/L, about 60 mg/L, about 65 mg/L, about 70 mg/L, about 75 mg/L, about 80 mg/L, about 85 mg/L, about 90 mg/L, about 95 mg/L, about 100 mg/L, about 150 mg/L, about 200 mg/L, about 250 mg/L, about 300 mg/L, about 350 mg/L, about 400 mg/L, about 450 mg/L, about 500 mg/L, about 550 mg/L, about 600 mg/L, about 650 mg/L, about 700 mg/L, about 750 mg/L, about 800 mg/L, about 850 mg/L, about 900 mg/L, about 950 mg/L, about 1,000 mg/L, about 1,100 mg/L, about 1,200 mg/L, about 1,300 mg/L, about 1,400 mg/L, about 1,500 mg/L, or the like. The free chlorine concentration of a chlorous acid aqueous solution can be any value within a range thereof.

In the present disclosure, the removal of microbes targets at least one selected from a virus, a microbe, and a eukaryote. Removal of microbes targets microorganisms that can cause food poisoning, pathogenic microorganisms, microorganism that can induce an infection, drug resistant bacteria (microbes), or the like.

In the present disclosure, the liquid for microbe-removal does not contain an animal derived component, an alcohol component, or a flavor.

The present disclosure provides a halal-compliant hand and finger liquid for microbe-removal, comprising 1.00% chlorous acid aqueous solution, 1.00% sodium dihydrogen phosphate, 1.00% potassium dihydrogen phosphate, and 97.00% ion exchange water, wherein chlorous acid (as HClO₂ = 68.46) is 400 ppm, and a free chlorine concentration (as Cl = 35.45) is 10 mg/L. The liquid for microbe-removal has a reduced chlorine odor upon use compared to conventional products while reliably maintaining the microbe-removing power of a chlorous acid aqueous solution with excellent user operability. The liquid for microbe-removal also has a deodorizing effect because the solution removes odor causing bacteria. The present liquid for microbe-removal does not contain an animal derived component, an alcohol component, or a flavor and is therefore halal-compliant. Microbes of hand and fingers can be removed conveniently by directly spraying onto the hand and fingers without a need for dilution. In this manner, a solution that is directly contacted with a hand and fingers for microbe removal while having a high microbe-removing power did not exist previously and is thus the world's first of a kind. The discovery that a liquid for microbe-removal comprising phosphate buffer, chlorous acid, and free chlorine with such concentrations is safe with regard to skin irritability, eye irritability, and skin sensitization while having a high microbe-removing power was unexpected.

The present disclosure provides a halal-compliant hand and finger liquid for microbe-removal, comprising 20.00% chlorous acid aqueous solution, 1.00% potassium dihydrogen phosphate, 1.00% dipotassium hydrogen phosphate, and 78.00% ion exchange water, wherein chlorous acid (as HClO₂ = 68.46) is 8,000 ppm, and a free chlorine concentration (as Cl = 35.45) is 200 mg/L. The present liquid for microbe-removal does not contain an animal derived component, an alcohol component, or a flavor and is therefore halal-compliant. The liquid for microbe-removal can be diluted (e.g., 4-fold dilution, 8-fold dilution, or 20-fold dilution) and used at a low cost at a processed food plant, large scale food preparation facility, or the like. In this manner, a solution that is directly contacted with a hand and fingers for microbe removal while having a high microbe-removing power did not exist previously and is thus the world's first of a kind. The discovery that a liquid for microbe-removal comprising phosphate buffer, chlorous acid, and free chlorine with such concentrations is safe with regard to skin irritability, eye irritability, and skin sensitization while having a high microbe-removing power was unexpected.

### (Chlorous acid aqueous solution and manufacturing example thereof)

A chlorous acid aqueous solution used in the present disclosure has a feature discovered by the inventors. A chlorous acid aqueous solution manufactured by any method such as a known manufacturing method described in the aforementioned document or the like can be used. As a representative example of composition, 61.40% chlorous acid aqueous solution, 1.00% potassium dihydrogen phosphate, 0.10% potassium hydroxide, and 37.50% purified water can be mixed and used (sold by the Applicant; 72 % chlorous acid aqueous solution corresponds to chlorous acid at 30000 ppm), but the composition is not limited thereto. The chlorous acid aqueous solution may also be 0.25%-75%, potassium dihydrogen phosphate may be 0.70%-17.42%, and potassium hydroxide may be 0.10%-5.60%. It is possible to use sodium dihydrogen phosphate instead of potassium dihydrogen phosphate, or sodium hydroxide instead of potassium hydroxide. As another representative example of the composition, 1.00% chlorous acid aqueous solution, 1.00% potassium dihydrogen phosphate, 1.00% sodium dihydrogen phosphate, and 97.00% purified water can be combined and used, but can be 0.1% to 5% chlorous acid aqueous solution, 0.68% to 13.6% potassium dihydrogen phosphate, and 0.60% to 12.0% sodium dihydrogen phosphate. As another representative example of the composition, 20.00% chlorous acid aqueous solution, 1.00% potassium dihydrogen phosphate, 1.00% dipotassium hydrogen phosphate, and 78.00% purified water can be mixed and used, but the composition can be 15% to 25% chlorous acid aqueous solution, 0.68% to 13.6% potassium dihydrogen phosphate, and 0.87% to 17.4% dipotassium hydrogen phosphate. While the decrease of chlorous acid due to contact with an organic matter under acidic conditions is attenuated for this agent, the germicidal effect is maintained. In addition, very little chlorine gas is generated. Further, the agent also has a feature of inhibiting amplification of odor from mixing chlorine with an organic matter.

In one embodiment, the chlorous acid aqueous solution of the present disclosure can be produced by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid.

Further, in another embodiment, the chlorous acid aqueous solution of the present disclosure can be produced from adding one compound from inorganic acids or inorganic acid salts, two or more types of compounds therefrom, or a combination thereof to an aqueous solution, in which chlorous acid is produced by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 3.2 to 8.5.

Furthermore, in another embodiment, the chlorous acid aqueous solution of the present disclosure can be produced from adding one compound from inorganic acids or inorganic acid salts or organic acids or organic acid salts, two or more types of compounds therefrom, or a combination thereof to an aqueous solution, in which chlorous acid is produced by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 3.2 to 8.5.

Further still, in another embodiment, the chlorous acid aqueous solution of the present disclosure can be produced from adding one compound from inorganic acids or inorganic acid salts or organic acids or organic acid salts, two or more types of compounds therefrom, or a combination thereof after adding one compound from inorganic acids or inorganic acid salts, two or more types of compounds therefrom, or a combination thereof to an aqueous solution, in which chlorous acid is produced by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 3.2 to 8.5.

Further, in another embodiment, carbonic acid, phosphoric acid, boric acid, or sulfuric acid can be used as the inorganic acid in the method described above.

Further still, in another embodiment, carbonate, inorganic hydroxide, phosphate, or borate can be used as the inorganic acid salt.

Further, in another embodiment, sodium carbonate, potassium carbonate, sodium bicarbonate, or potassium bicarbonate can be used as the carbonate.

Furthermore, in another embodiment, sodium hydroxide, potassium hydroxide, calcium hydroxide, or barium hydroxide can be used as the inorganic hydroxide.

Further still, in another embodiment, disodium hydrogen phosphate, sodium dihydrogen phosphate, trisodium phosphate, tripotassium phosphate, dipotassium hydrogen phosphate, or potassium dihydrogen phosphate can be used as the phosphate.

Further, in another embodiment, sodium borate or potassium borate can be used as the borate.

Furthermore, in another embodiment, succinic acid, citric acid, malic acid, acetic acid, or lactic acid can be used as the organic acid.

Further still, in another embodiment, sodium succinate, potassium succinate, sodium citrate, potassium citrate, sodium malate, potassium malate, sodium acetate, potassium acetate, sodium lactate, potassium lactate, or calcium lactate can be used as the organic acid salt.

In a method of manufacturing an aqueous solution comprising chlorous acid (HClO₂) that can be used as a germicide (chlorous acid aqueous solution), chlorous acid (HClO₂) is produced by adding hydrogen peroxide (H₂O₂) in an amount required to produce chlorous acid by a reducing reaction of chloric acid (HClO₃) obtained by adding sulfuric acid (H₂SO₄) or an aqueous solution thereof to an aqueous solution of sodium chlorate (NaClO₃) so that the aqueous solution is in an acidic condition. The basic chemical reaction of this method of manufacturing is represented by the following formula A and formula B.

### [Chemical Formula 4]

**2NaClO₃** + **H₂SO₄** → **2HClO₃** + **Na₂SO₄↓** **(formula A)**

**HClO₃** + **H₂O₂ → HClO₂ + H₂O** + **O₂↑** **(formula B)**

Formula A indicates that chloric acid is obtained by adding sulfuric acid (H₂SO₄) or an aqueous solution thereof at an amount and concentration where the pH value of an aqueous sodium chlorate (NaClO₃) solution can be maintained within acidity. Next, formula B indicates that chloric acid (HClO₃) is reduced by hydrogen peroxide (H₂O₂) to produce chlorous acid (HClO₂) .

### [Chemical Formula 5]

HClO₃+H₂O₂ → 2ClO₂+H₂O+O₂T↑ (formula C)

2ClO₂+H₂O₂ → 2 HClO₂+O₂↑ (formula D)

2 ClO₂+H₂O ⇔ HClO₂+HClO₃ (formula E)

2 HClO₂⇔H₂O+CL₂O₃ (formula F)

At this time, chlorine dioxide gas (ClO₂) is generated (formula C). However, coexistence with hydrogen peroxide (H₂O₂) produces chlorous acid (HClO₂) through the reactions in formulas D to F.

Meanwhile, the produced chlorous acid (HClO₂) has a property of being decomposed early into chlorine dioxide gas or chlorine gas due to the presence of chloride ion (Cl⁻), hypochlorous acid (HClO), and other reduction product, or resulting in a decomposition reaction among a plurality of chlorous acid molecules with one another. Thus, it is necessary to be prepared so that chlorous acid (HClO₂) can be sustained for an extended period of time in order to be useful as a germicide.

In this regard, chlorous acid (HClO₂) can be stably sustained over an extended period of time from creating a transition state to delay a decomposition reaction by adding one compound from inorganic acids, inorganic acid salts, organic acids, or organic acid salts, two or more types of compounds therefrom, or a combination thereof to the chlorous acid (HClO₂) or chlorine dioxide gas (ClO₂) obtained by the method described above or an aqueous solution containing them.

In one embodiment, it is possible to utilize a mixture prepared from adding one compound from inorganic acids or inorganic acid salts, specifically carbonate or inorganic hydroxides, two or more types of compounds therefrom, or a combination thereof to the chlorous acid (HClO₂) or chlorine dioxide gas (ClO₂) obtained by the method described above or an aqueous solution containing them.

In another embodiment, it is possible to utilize a mixture prepared from adding one compound from inorganic acids, inorganic acid salts, organic acids, or organic acid salts, two or more types of compounds therefrom, or a combination thereof to an aqueous solution to which one compound from inorganic acids or inorganic acid salts, specifically carbonate or inorganic hydroxide, two or more types of compounds therefrom, or a combination thereof is added.

Additionally, in another embodiment, it is possible to utilize a mixture prepared from adding one compound from inorganic acids or inorganic acid salts or organic acids or organic acid salts, two or more types of compounds therefrom, or a combination thereof to the aqueous solution manufactured by the method described above.

Examples of the inorganic acid described above include carbonic acid, phosphoric acid, boric acid, and sulfuric acid. Examples of the inorganic acid salt include phosphate and borate, in addition to carbonate and inorganic hydroxide. Specifically, sodium carbonate, potassium carbonate, sodium bicarbonate, or potassium bicarbonate works well in use as the carbonate; sodium hydroxide, potassium hydroxide, calcium hydroxide, or barium hydroxide works well in use as the inorganic hydroxide; disodium hydrogen phosphate, sodium dihydrogen phosphate, trisodium phosphate, tripotassium phosphate, dipotassium hydrogen phosphate, or potassium dihydrogen phosphate works well in use as the phosphate; and sodium borate or potassium borate works well in use as the borate. Examples of the organic acid include succinic acid, citric acid, malic acid, acetic acid, and lactic acid. Further, sodium succinate, potassium succinate, sodium citrate, potassium citrate, sodium malate, potassium malate, sodium acetate, potassium acetate, sodium lactate, potassium lactate, or calcium lactate is suitable as the organic acid salt.

When an acid and/or a salt thereof is added, a transition state, such as Na⁺ + ClO₂⁻ <-> Na-ClO₂, K⁺ + ClO₂⁻<-> K-ClO₂, or H⁺ + ClO₂⁻ <-> H-ClO₂ can be temporarily created to delay the progression of chlorous acid (HClO₂) to chlorine dioxide (ClO₂), which enables the manufacture of an aqueous solution comprising chlorous acid (HClO₂) that sustains chlorous acid (HClO₂) for an extended period of time and generates a low amount of chlorine dioxide (ClO₂) .

The following represents the decomposition of chlorite in an acidic solution.

### [Chemical Formula 6]

5ClO₂⁻+4H⁺→4ClO₂+5Cl⁻+2H₂O (a)

(5NaClO₂+4CH₃COOH

→4ClO₂+4CH₃COONa+NaCl+2H₂O)

3ClO₂⁻→2ClO₃⁻+Cl⁻ (b)

(3NaClO₂→2NaClO₃+NaCl) Autodecomposition

ClO₂⁻→Cl⁻+2O (c)

As expressed in the formula, the rate of decomposition of an aqueous chlorite solution is greater when pH is lower, i.e., more acidic. Specifically, the absolute rates of reactions (a), (b), and (c) in the formula described above increase. For example, although the ratio accounted for by reaction (a) decreases when pH is lower, the total decomposition rate changes significantly, i.e., to a larger value. Thus, the amount of generated chlorine dioxide (ClO₂) also increases with the decrease in pH. Thus, a lower pH value results in faster disinfection or bleaching. However, irritable and harmful chlorine dioxide gas (ClO₂) renders usage more difficult and negatively affects the health of a human being. Further, a reaction of chlorous acid to chlorine dioxide progresses quicker to render chlorous acid unstable. In addition, the period during which a germicidal power can be sustained is very short.

When the inorganic acids, inorganic acid salts, organic acids, or organic acid salts described above are added to an aqueous solution comprising chlorous acid (HClO₂), pH values are adjusted in the range of 3.2 to 8.5 from the viewpoint of balancing suppression of the generation of chlorine dioxide and germicidal power.

### (Method of use of liquid for microbe-removal)

The liquid for microbe-removal of the present disclosure can remove microbes of an animal by direct contact with an animal. Microbe removing methods include a rubbing method, scrubbing method, and basin method. A swabbing method can also be used. The microbes can be removed upon entry into food processing plants, restaurant facilities including diners, food preparation facilities including kitchens, etc.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The above descriptions and the following Examples are not provided to limit the present invention, but for the sole purpose of exemplification. Thus, the scope of the present invention is not limited to the embodiments and Examples specifically described herein and is limited only by the scope of claims.

### [Examples]

When necessary, humans used in the following Examples were handled based on the Declaration of Helsinki. Animals were handled in compliance with the principles of 3R as needed under the spirit of animal welfare. While the specific products described in the Examples were used, reagents can be substituted with an equivalent product from another manufacturer (Sigma, Wako Pure Chemical, Nacalai Tesque, etc.).

### (Quantification Method of Chlorous Acid Aqueous Solution)

About 5 g of the present product is precisely measured. Water is added thereto so that the solution is exactly 100 ml. After 20 ml of the sample solution is accurately measured, placed in an iodine flask, and combined with 10 ml of sulfuric acid (1→10) 1 g of potassium iodide is added thereto. The flask is immediately sealed and shaken well. A potassium iodide reagent is poured into the top portion of the iodine flask and left standing in the dark for 15 minutes. The plug is then loosened to pour in a potassium iodide reagent and the flask is sealed immediately, shaken well, and mixed. Thereafter,, free iodine is titrated with 0.1 mol/L sodium thiosulfate (indicator: starch reagent). The indicator is added after the color of the solution has changed to a light yellow color. A blank test is separately conducted for correction (1 mL of 0.1 mol/L sodium thiosulfate solution = 1.711 mg of HClO₂).

### (Manufacturing Examples)

Chlorous acid aqueous solution formulations used in the following Examples were manufactured as follows. Chlorous acid aqueous solution may be abbreviated as "CAAS" herein, but the terms are synonymous.

### Component analysis table for chlorous acid aqueous solution

**[Table 1]**

| CAAS specification | Specification Value | Match/Not a Match |
|---|---|---|
| Content | 4-6 % or higher | 4.1% |
| Attribute | light yellowish green to yellowish red | yellowish red |
| Confirmation Test (1) | When 0.1 ml of aqueous potassium permanganate solution (1 → 300) is added to 5 ml of an aqueous solution of the present product (1→20), the solution turns reddish purple. When 1 ml of sulfuric acid (1→ 20) is added thereto, the solution turns light yellow. | Match |
| Confirmation Test (2) | An aqueous solution of the present product (1_{→}20) has maximum absorbance sections at wavelengths 258-262 nm and 346-361 nm. | Match |
| Confirmation Test (3) | If potassium iodide starch paper is dipped in the present product, the potassium iodide starch paper changes to a blue color and then the color fades. | Match |
| Purity Test (1) | 1.0 µg/g or lower for lead | Below detectable limit |
| Purity Test (2) | 1.0 µg/g or lower for Ag₂O₃ | Below detectable limit |

A chlorous acid aqueous solution formulation was manufactured using this chlorous acid aqueous solution based on the following composition.

**[Table 2]**

| | Raw **material** | Blended Amount | Concentration | Acceptable Range |
|---|---|---|---|---|
| 1 | Tap water | 258.0 g | | |
| 2 | Dipotassium hydrogen phosphate | 17.0 g | 1.70% | 0.70%-13.90% |
| 3 | Potassium hydroxide | 5.0 g | 0.50% | 0.10%-5.60 % |
| 4 | Chlorous acid aqueous solution (pH 3.5) | 720.0 g | 72.00% | 0.25%-75% |
| Total | | Chlorous acid 30000 ppm | 1000 g | |

**[Table 3]**

| CAAS specification | chlorous acid aqueous solution formulation manufactured with chlorous acid aqueous solution |
|---|---|
| content | 3.0% |
| Attribute | yellow |
| Confirmation Test (1) | Match |
| Confirmation Test (2) | Match |
| Confirmation Test (3) | Match |
| Purity Test (1) | Below detectable limit |
| Purity Test (2) | Below detectable limit |

For the "chlorous acid aqueous solution formulation manufactured with chlorous acid aqueous solution" prepared based on the preparation method described above, the concentration of "chlorous acid aqueous solution" was measured based on the "Quantification method of chlorous acid aqueous solution" described above, and the buffer prepared to achieve the free chlorine concentration described in each Example (phosphate buffer comprising dipotassium hydrogen phosphate and potassium dihydrogen phosphate) was used to prepare the chlorous acid aqueous solution of each Example.

### (Example 1-1)

### (Microbe removing effect)

The following were used:
Bacterial strain: Escherichia coli NBRC3972 (used own bacteria strain)
Medium: desoxycholate agar medium
Dilution (retrieval): sterilized water, saline, sterilized vinyl gloves
Drug solution concentration: α) free chlorine concentration (as Cl = 35.45): 25 mg/L or higher; 3 mL × 2 (dispenser), disinfection (microbe removal) time of 2 minutes β) free chlorine concentration (as Cl = 35.45): 10 mg/L or higher; 3 mL × 2 (dispenser), disinfection (microbe removal) time of 5 minutes γ) free chlorine concentration (as Cl = 35.45): 10 mg/L or higher: 3 mL × 2 (dispenser), disinfection (microbe removal) time of 2 minutes

### (Test procedures)

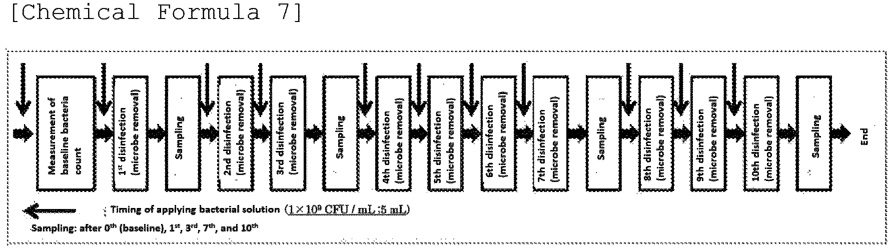

(1) 3 mL of indicator bacterial solution is applied to the hand and fingers (one hand) and dried for 2 minutes.
(2) A vinyl glove is worn. 50 mL of sterilized water is poured in, and the hand is rubbed so that the sterilized water spreads to the entire hand (the entrance is closed so that the sterilized water would not spill).
(3) Sterilized water is collected. 0.1 mL of solution diluted with saline is smeared onto a medium at an appropriate time and cultured at 35°C (baseline).
(4) After the step of (1), the hand and fingers (one hand) is disinfected while rubbing it in for 2 minutes (5 minutes in test segment β).
(5) The step of (4) is repeated 10 times, and the step of (2) is performed after the 1^{st}, 3^{rd}, 7^{th}, and 10^{th} disinfection.

### <Determination criteria>

≥ 2Log10 reduction within 5 minutes after the 1st disinfection, and ≥ 3Log10 reduction within 5 minutes after the 10th disinfection

### (Results)

### Drug solution concentration:

### [Chemical Formula 8]

| Set value | Actual measurement value | |
|---|---|---|
| | Free chlorine concentration (as Cl = 35.45) | Content: chlorous acid (as HClO₂ = 68.46) |
| Free chlorine concentration (as Cl = 35.45): 25 mg/L | 22 mg/L | 770 ppm |
| Free chlorine concentration (as Cl = 35.45): 10 mg/L | 10 mg/L | 325 ppm |

| unit : Log₁₀ (CFU / mL) | | | | | | |
|---|---|---|---|---|---|---|
| Number of disinfections (contacts) | α) Free chlorine concentration 25 mg/L, 2 min | | β) Free chlorine concentration 10 mg/L, 5 min | | γ) Free chlorine concentration 10 mg/L, 2 min | |
| | Viable bacteria count | Number of reduced bacteria | Viable bacteria count | Number of reduced bacteria | Viable bacteria count | Number of reduced bacteria |
| 0(baseline) | 5.64^{∗1} | - | 5.64^{∗1} | - | 5.64^{∗1} | - |
| 1 | < 1.00 | > 4.6 | 2.26 | 3.4 | 4.69 | 1.0 |
| 3 | < 1.00 | > 4.6 | < 1.00 | > 4.6 | 4.62 | 1.0 |
| 7 | < 1.00 | > 4.6 | < 1.00 | > 4.6 | 2.49 | 3.2 |
| 10 | < 1.00 | > 4.6 | < 1.00 | > 4.6 | 2.49 | 3.2 |

| | | | | | | |
|---|---|---|---|---|---|---|
| *1: Mean value after 3 disinfections (estimated mean adhering hand and finger contaminating bacteria count was 7 Log (x 50 dilution) | | | | | | |

### Results from a free chlorine concentration (as Cl = 35.45) of 25 mg/L and 6 mL (3 mL/hand x twice)

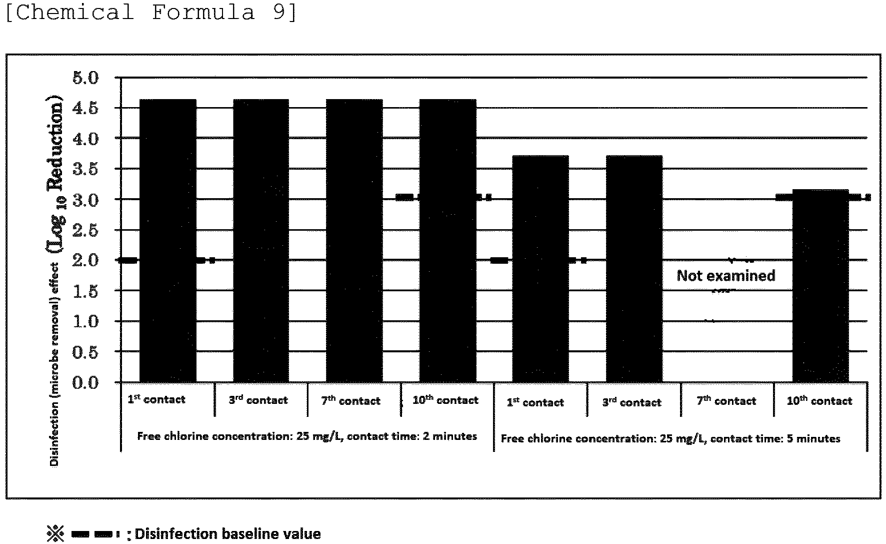

### Results from a free chlorine concentration (as Cl = 35.45) of 10 mg/L and 6 mL (3 mL/hand × twice)

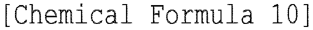

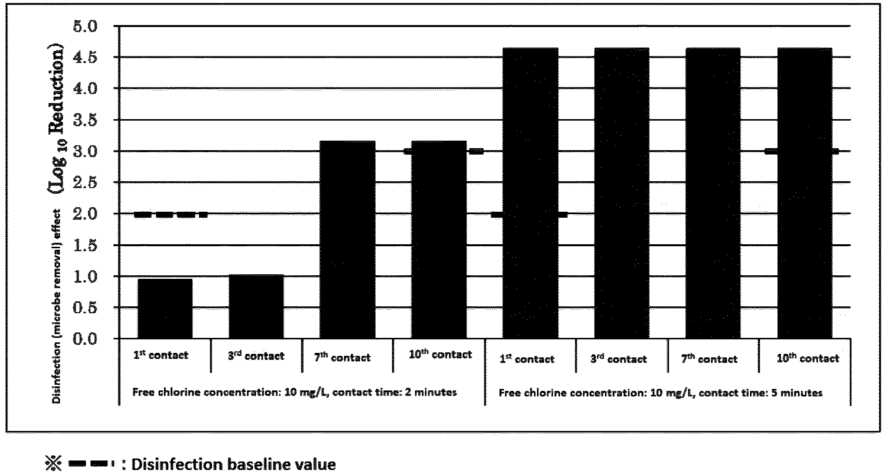

When a test was conducted by using a diluent of chlorous acid aqueous solution with a free chlorine concentration (as Cl = 35.45) of 25 mg/l and reducing the contact time from 5 minutes to 2 minutes at 3 ml/hand × twice, a sterilization effect of 3 Log or greater was observed from the 1^{st} disinfection, and a sterilizing effect of 3 Log or greater was able to be sustained to the 10^{th} disinfection in the same manner as a test with a contact time of 5 minutes.

Disinfection (microbe-removal) was applied at 3 ml/hand × twice by using a diluent of chlorous acid aqueous solution with a free chlorine concentration (as Cl = 35.45) of 10 mg/l. If the contact time was 5 minutes, a sterilization effect of 3 Log or greater was observed from the 1^{st} disinfection, and a sterilization effect of 3 Log or greater was sustained to the 10^{th} disinfection. However, if the contact time was 2 minutes, a sterilization effect of 2 Log of greater, which is the baseline value, was observed on the 1^{st} disinfection. For this reason, it was found that a sufficient sterilization effect was not achieved with a contact time of 2 minutes when disinfection (microbe removal) was applied at 3 ml/hand × twice by using a diluent of chlorous acid aqueous solution with a free chlorine concentration (as Cl = 35.45) of 10 mg/l. It was found, however, that the bacterial count gradually decreased with repeated contact. A sterilizing effect of 3log of greater was observed from the 1^{st} disinfection even with a diluent of chlorous acid aqueous solution with a free chlorine concentration (as Cl = 35.45) of 10 mg/l if the contact time was 5 minutes.

It is demonstrated that chlorous acid aqueous solution formulations meet the determination criteria based on the ASTEM Standard E1174 evaluation criteria and have a sufficient microbe-removing effect at the two set values (free chlorine concentration of 25 mg/L, content as HClO₂ of 1,000 ppm; and free chlorine concentration of 10 mg/L, content as HClO₂ of 400 ppm).

The sterilizing effect was studied using a rubbing method (waterless method) which is one of the hand and finger disinfecting (microbe-removing) method during surgery at a hospital as the test condition. However, a method of using a hand and finger disinfecting (microbe-removing) agent installed at the entrance/exit of a facility with a low dose of agent is generally used through a method described below (single dose dispensed by a dispenser is 3 ml/dose). 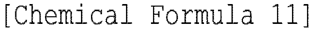 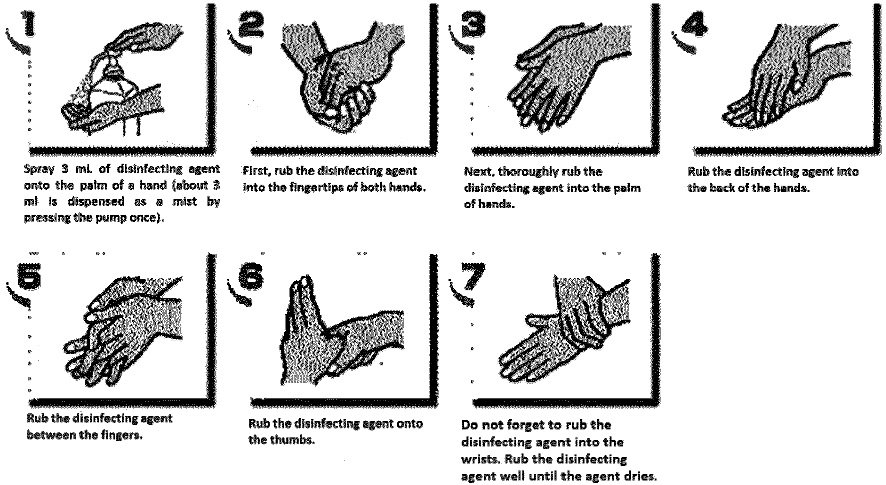 ("Method of using hand and finger disinfecting (microbe-removing) agent" (see https://www.yoshida-homecare.com/jitsurei/01.html)

Thus, it was examined whether a sterilizing effect of 3Log or greater is observed even with a single dose of agent dispensed by a dispenser (3.5 ml) at a free chlorine concentration (as Cl = 35.45) of 10 mg/L.

### (Example 1-2)

The following were used:
Bacterial strain: Escherichia coli NBRC3972 (used own bacteria strain)
Medium: desoxycholate agar medium
Dilution (retrieval): sterilized water, saline, sterilized vinyl gloves
Drug solution concentration: α) free chlorine concentration (as Cl = 35.45): 10 mg/L or higher; 3 mL × 1 (dispenser), disinfection (microbe-removal) time of 5 minutes

### (Test procedures)

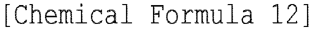

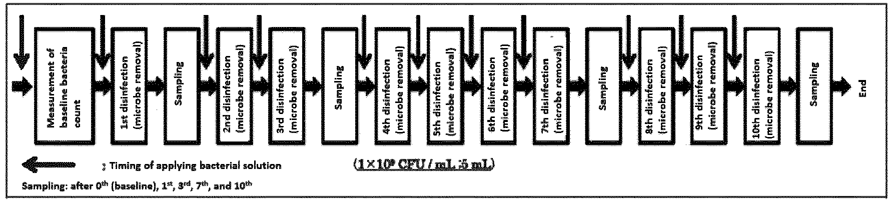

(1) 3 mL of indicator bacterial solution is applied to the hand and fingers (one hand) and dried for 2 minutes.
(2) A vinyl glove is worn. 50 mL of sterilized water is poured in, and the hand is rubbed so that the sterilized water spreads to the entire hand (the entrance is closed so that the sterilized water would not spill).
(3) Sterilized water is collected. 0.1 mL of the solution diluted with saline is smeared onto a medium at an appropriate time and cultured at 35°C (baseline).
(4) After the step of (1), the hands and fingers (both hands) are disinfected (microbes removed) while rubbing it in for 5 minutes in accordance with the "Method of using hand and finger disinfecting (microbe-removing) agent" described above.
(5) The step of (4) is repeated 10 times, and the step of (2) is performed after the 1^{st}, 3^{rd}, 7^{th}, and 10^{th} disinfection.

### <Determination criteria>

≥ 2Log10 reduction within 5 minutes after the 1^{st} disinfection (microbe removal), and ≥ 3Log10 reduction within 5 minutes after the 10th disinfection (microbe removal)

### Drug solution concentration:

### [Chemical Formula 13]

| Set value | Actual measurement value | |
|---|---|---|
| | Free chlorine concentration (as Cl = 35.45) | Content: chlorous acid (as HClO₂ = 68.46) |
| Free chlorine concentration (as Cl = 35.45): 10 mg/L | 12.7 mg/L | 350 ppm |

| Number of disinfections (contacts) | α) Free chlorine concentration (as Cl = 35.43): 10 mg/L | |
|---|---|---|
| | Viable bacteria count | Number of reduced bacteria |
| **0 (baseline)** | 6.18 | - |
| 1 | 2.45 | 3.73 |
| 3 | 1.00 | 5.18 |
| 7 | 1.70 | 4.48 |
| 10 | 2.08 | 4.10 |

| | | |
|---|---|---|
| **Unit:** : Log₁₀ (CFU / mL) *estimated mean adhering hand and finger contaminating bacteria count was 8 Log (x 50 dilution) | | |

### Results from a free chlorine concentration (as Cl = 35.45) of 10 mg/L and 3 mL (3 mL/hand × once)

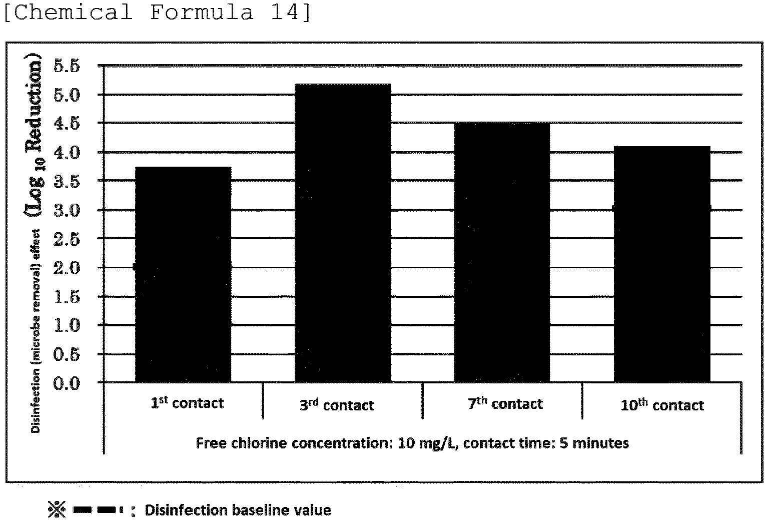

A diluent of chlorous acid aqueous solution with a free chlorine concentration (as Cl = 35.45) of 10 mg/L was used on the hand and fingers (both hands) at 3 ml/hand × once for test with a contact time of 5 minutes in accordance with the "Method of using hand and finger disinfecting (microbe-removing) agent" described above. As a result, a sterilizing effect of 2Log of greater was observed from the first contact, and a sterilization effect of 3 Log or greater was sustained to the 10^{th} disinfection. For this reason, it was found that a sterilization effect of 3Log or greater was achieved from the first contact in accordance with "*1 Method of using hand and finger disinfecting (microbe-removing) agent" even at a drug dose of 3 ml/hand × once if the contact time is 5 minutes using a diluent of chlorous acid aqueous solution with a free chlorine concentration (as Cl = 35.45) of 10 mg/L.

### Results from a free chlorine concentration (as Cl = 35.45) of 10 mg/L and 6 mL (3 mL/hand × twice)

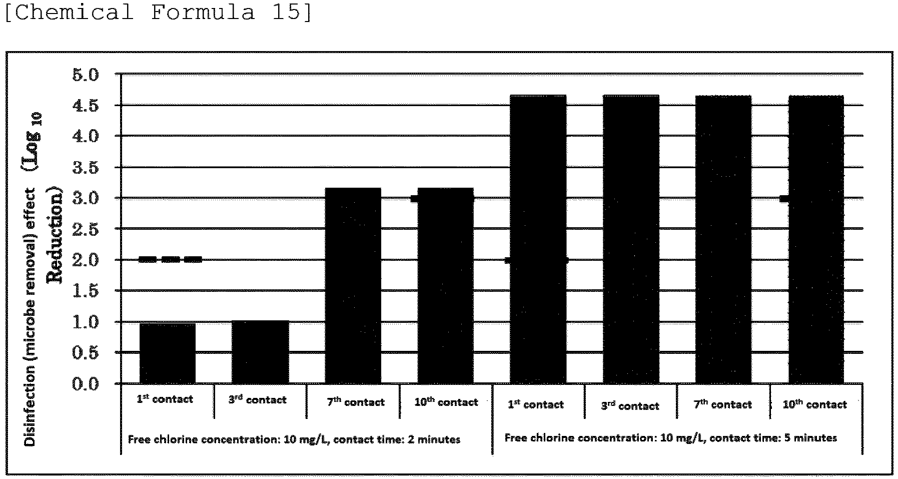

### Results from a free chlorine concentration (as Cl = 35.45) of 10 mg/L and 3 mL (3 mL/hand × once)

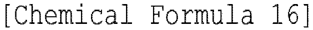

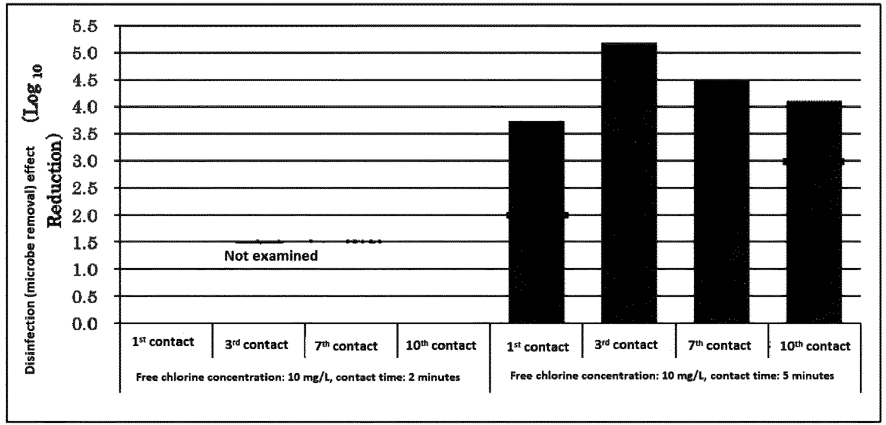

In view of the above results, it was found that a sufficient sterilization effect is achieved with a contact time of 5 minutes when a diluent of chlorous acid aqueous solution with a free chlorine concentration (as Cl = 35.45) of 10 mg/l is dispensed on the palm of hands at 3 ml/hand × once using a dispenser in accordance with the "*1 Method of using hand and finger disinfecting (microbe-removing) agent". Since a sufficient sterilization effect was not achieved with a contact time of 2 minutes at 3 ml/hand × twice of chlorous acid aqueous solution with a free chlorine concentration (as Cl = 35.45) of 10 mg/l, it was found that the immediate effect did not improve by increasing the amount of drug solution.

In this regard, sterilization effect confirmation tests were conducted by using this result in accordance with ASTE M1174 Method (draft) by purchasing Serratia marcescens ATCC14756 (corresponding to NBRC 12648) for (1) treatment in according with "*1 Method of using hand and finger disinfecting (microbe-removing) agent" for a contact time of 2 minutes of a chlorous acid aqueous solution with a free chlorine concentration (as Cl = 35.45) of 25 mg/L at 3 ml/hand × once and (2) treatment in according with "Method of using hand and finger disinfecting (microbe-removing) agent" for a contact time of 5 minutes of a chlorous acid aqueous solution with a free chlorine concentration (as Cl = 35.45) of 10 mg/L at 3 ml/hand × once.

### (Example 2)

### (Skin irritability test on chlorous acid aqueous solution formulation using rabbits)

To study the skin irritability and corrosiveness of a chlorous acid aqueous solution formulation, a skin irritability test was conducted using three female Kbl:NZW rabbits. The irritability reaction was compared by setting a three rabbit sodium hypochlorite formulation treatment group as a comparative control group. 0.5 mL of test substance was applied homogenously to a lint patch and exposed 4 hours to the skin on the back of the rabbits. The reaction of the skin and the general condition were observed at 1, 24, 48, and 72 hours, and every day up to day 14 after patch removal. In addition, the body weight before administration and at the end of observation was measured.

The results were as follows.

A reaction of the skin was not observed at the sites of administration of any animal during the observation period with a chlorous acid aqueous solution formulation. As for the GHS hazard category (skin corrosion and skin irritability), a reaction of the skin was not exhibited during the observation period, and the calculated mean score did not reach the lower limit value of any of the categories, so that this was classified as not falling under the categories.

Meanwhile, grade 1 erythema and grade 1 or 2 edema were found at a site of administration in all three cases from observation at 1 hour after patch removal with a sodium hypochlorite formulation. These reactions were subsequently exacerbated so that grade 3 erythema and edema were exhibited at 72 hours after patch removal. While edema exhibited a trend of recovery 5 days after patch removal, exacerbation of erythema to grade 4 was found 5 or 7 days after patch removal in two cases. Grade 2 or 3 erythema was observed 14 days after patch removal (final observation date) in all cases. Exfoliation was observed 5 or 6 days after patch removal, and a scar was found on day 9 in one case. At 14 days after patch removal, a scar was found in one case, and exfoliation was found in two cases. As for the GHS hazard category (skin corrosion and skin irritability), a reaction of the skin (irritability) did not reverse in all cases during the observation period, and a scar that did not recover in 14 days after patch removal determined as a corrosive reaction was observed in one case. Thus, this was classified as category 1 (corrosive). While exposure of 4 hours or less was not conducted, the subcategory in category 1 was predicted to be 1B or 1C.

An abnormality in the general condition was not found, and the change in body weight was also within the normal range in all 6 rabbits during the observation period.

It was determined in view of the above results that a chlorous acid aqueous solution formulation does not have a corrosive and irritable action on rabbit skin under the test conditions (GHS classification: not falling under the categories). Meanwhile, it was determined that a sodium hypochlorite formulation has a corrosive action on rabbit skin (GHS classification: category 1).

### 1. Title

### Skin irritability test on chlorous acid aqueous solution formulation using rabbits

### 2. Test objective

To study the skin irritability and corrosiveness of a chlorous acid aqueous solution formulation, a skin irritability test is conducted using rabbits. A sodium hypochlorite formulation is administered as a comparative control to compare the irritability reactions thereof.

### 3. GLP complied

-"Ministerial Ordinance Concerning the Standards for the Conduct of Non-clinical Studies on the Safety of Drugs" (ordinance of the Ministry of Health and Welfare No. 21, dated March 26, 1997)"

### 4. Referenced guideline

- OECD Guideline for Testing of Chemicals 404 (28 July 2015: Acute Dermal Irritation/Corrosion)

### 5. Complied animal experiment related regulations

This test was conducted in compliance with "Act on Welfare and Management of Animals" (October 1, 1973, Act No. 105, final revision: May 30, 2014, Act No. 46) and "Standards Relating to the Care and Management of Laboratory Animals and Relief of Pain" (Notice No. 88 of the Ministry of Environment dated April 28, 2006, final revision: Notice No. 84 of the Ministry of Environment dated August 30, 2013). The test was examined and approved prior to the start of the test by the animal experiment committee of the BioSafety Research Center (approval no. 17-0030A) and conducted in accordance with the animal ethics evaluation criteria specified in the "Guidelines for animal experiments" (June 2, 2014) of the BioSafety Research Center.

### 13.1. Test substance

### 13.1.1 Name

### Chlorous acid water (formulation)

13.1.5 Component content
13.1.5.1. Active ingredient
0.8% chlorous acid
13.1.5.1.1. Active ingredient (actual measurement value)

Chlorous acid aqueous solution (total chlorine content:
HClO₂ = 68.46) [%] 0.84
Free chlorine (HClO₂ = 68.46) [mg/L] 448

### 13.1.5.2. Other components

Dipotassium hydrogen phosphate
Potassium dihydrogen phosphate

### 13.1.6. Stability

Stable under designated conditions until the expiration date

### 13.1.7. Condition of substance

### 13.1.7.1. Outer appearance

Bright yellow to light greenish yellow and clear liquid 13.1.7.2. Odor
Chlorine odor

### 13.1.8. Expiration date

February 7, 2018 (one year after manufacture)

### 13.1.9. Storage condition

### Refrigerated away from light and sealed

### 13.1.9.1. Actual measurement value of storage temperature and storage period

- 3.1 to 7.5°C
- May 2, 2017 to June 13, 2017 (test substance: date of receipt to date of use)

### 13.1.10. Precaution for use

Wear a mask/glove

### 13.1.11. Treatment of residual test substance

### Disposed in entirety

### 13.2. Comparative control substance

### 13.2.1. Name

Sodium hypochlorite solution (formulation)

### 13.2.2. Production name

Name: Purelox-10

### 13.2.3. Application

Pharmaceutical product

### 13.2.4. Lot number

6511

### 13.2.5. Component content

### 13.2.5.1. Active ingredient

10% sodium hypochlorite

### 13.2.5.2. Other components

Unknown

### 13.2.6. Stability

Stable under designated conditions until the expiration date

### 13.2.7. Condition of substance

### 13.2.7.1. Outer appearance

Light yellow clear liquid

### 13.2.7.2. Odor

Chlorine odor

### 13.2.8. Expiration date

February 2018

### 13.2.9. Storage condition

Refrigerated away from light and sealed

### 13.2.9.1. Actual measurement value of storage temperature and storage period

- 3.1 to 7.5°C
- May 2, 2017 to June 13, 2017 (comparative control substance: date of receipt to date of use)

### 13.2.10. Precaution for use

Wear a mask/glove

### 13.2.11. Treatment of residual comparative control substance

The residual comparative control substance distributed from the manager of the test substance, etc. for the test was disposed.

### 14. Test material and method

### 14.1. Tested animal

### 14.1.1. Species

Rabbit

### 14.1.2. Line [grade]

New Zealand white species (Kbl:NZW) [SPF]

### 14.1.3. Producer

Kitayama Labes Co., Ltd.

### 14.1.4. Distributor

Oriental Yeast Co., Ltd.

### 14.1.5. Reason for selecting the test system

As the animal, white rabbits recommended by the guidelines were selected. As the line, New Zealand white species that are commonly used in this type of test were selected.

### 14.1.6. Age

16-week old animals were purchased, and a test substance was administered at 17 weeks old (body weight range: 2.89 to 3.20 kg).

### 14.1.7. Number of animals purchased

7 females

### 14.1.8. Number of animals used

6 females

### 14.2. Rearing management

### 14.2.1. Rearing environment

Animals were reared in rearing room No. 7-207 [positive pressure] (W 4.8 × D 10.3 × H 2.6 m), and the reference values for controlling the environment were the following. Temperature: 17 to 23°C (actual measurement value: 20.8 to 21.4°C)
Humidity: 35 to 70% RH (actual measurement value: 50 to 67% RH)
Number of air ventilations: 8 or more/h
Lighting: 12 hours (turn on at 7:00, turn off at 19:00)

One animal was housed in each rearing cage (W 62.2 × D 75.6 × H 46.2 cm). A rearing cage was exchanged at a frequency of every other week, and a feeder was exchanged at a frequency of once a week.

### 14.2.2. Feed

Solid feed (RC4, Lot No. 161111, Oriental Yeast Co., Ltd.) was made freely available from a feeder.

Test results (AR-16-JP-114796-01-JA) associated with contaminants in the feed used were obtained from the manufacturer to confirm that the value was within the proposed permissible reference value of the Japan Experimental Animal Food Association.

### 14.2.3. Water supply

Tap water was made freely available from an automatic water supplying nozzle.

Water quality testing based on the Waterworks Act was commissioned to an external agency in April 2017. The test results were confirmed to be within the reference value for waterworks quality standards (Result No. K17-0031). In addition to the water quality test described above, bacteria test (general bacteria and *E. coli* test) was conducted at the BioSafety Research Center to confirm that bacteria is not detected in May, June, and July of 2017 (No. GT17-05, No. GT17-06, No. GT17-07).

### 14.3. Quarantine/acclimation

The quarantine period was from delivery of the animals to measurement of body weight on the day before administration, and the acclimation period was up to before administration.

During this period, the general condition was observed once a day (excluding Saturdays and Sundays), and the body weight was measured upon delivery and day before administration (before shaving).

### 14.4. Individual identification

The serial number described on the auricle of animals by Kitayama Labes Co., Ltd. was used as the animal number (Animal ID No.), and the animal ID card explicitly describing the animal number was appended to the cage to identify the animals.

### 14.5. Selection of animals

The hair on the back of the torso of all animals was shaved with an electrical clipper (replacement blade: 0.1 mm) about 24 hours before administration. On the day of administration, each animal was confirmed to have no abnormality in the general condition and change in body weight during the quarantine/acclimation period. Since an injury or scar was found at an expected site of administration on the shaved skin of one animal (animal number 5), the animal was excluded from the test (surplus animal), and the remaining 6 animals were selected as the test animals.

### 14.6. Handling of surplus animals and tested animals after completion of observation

Surplus animals were transferred to the animal management department.

Tested animals were euthanized by pentobarbital sodium anesthesia.

### 14.7. Constitution of test group and reason for selecting test method

### 14.7.1. Constitution of test group

### [Chemical Formula 17]

| Test group | Number of animals | Animal number | Exposure time |
|---|---|---|---|
| Test substance treatment group | 3 | 1, 2, 3 | 4 hours |
| Comparative control substance treatment group | 3 | 4, 6, 7 | |

### 14.7.2. Reason for selecting test method

In view of the information of a chlorous acid aqueous solution lacking irritability on the skin, a method of exposing a total of three rabbits to a test substance for 4 hours was selected in accordance with the test guidelines. The package insert of the product did not have a description suggesting corrosiveness for sodium hypochlorite formulations.

### 14.8. Preparation of test substance

The provided test substance was directly used for administration.

### 14.9. Administration method

For administration, 0.5 mL of test substance was spread homogeneously on the lint surface of a 2.45 × 2.45 cm patch (lint cloth, Hasegawa Menkou) by using a MICROMAN (model M1000, Gilson), and then applied to the back skin of animals using a paper adhesive tape (KENZ). Furthermore, the entire treatment site was covered with a large lint cloth (Hasegawa Menkou), which was secured with an adhesive bandage (New-HALE, Hasegawa Menkou). Exposure time was 4 hours. The patch was removed after completion of exposure. The site of administration was marked with a permanent marker and wiped with injection water and tissue paper.

15. Observation, measurement, and testing

### 15.1. Observation of skin reaction

Erythema, eschar formation, and edema at sites of administration were observed 1, 24, 48, and 72 hours after patch removal, and scored in accordance with determination criteria for skin reactions (Draize [1], 1959). For animals found to not have a skin reaction 72 hours after patch removal, observation was ended at that point. Animals found to have a skin reaction even at 72 hours after patch removal were observed once a day until recovery from the next day, with 14 days after patch removal as the longest.

Sites of administration for all cases were captured as reference data using a digital camera upon observation at 24 hours after patch removal.

### Determination criteria for skin reaction

### [Chemical Formula 18]

| Degree of skin irritating reaction | Grade |
|---|---|
| 1) Erythema and eschar formation Maximum grade of 4 | |
| No erythema | 0 |
| Very mild erythema (barely recognizable) | 1 |
| Clear erythema | 2 |
| Moderate or severe erythema | 3 |
| Severe erythema (dark red color) or eschar formation (erythema ungradable) | 4 |
| 2) Edema formation Maximum grade of 4 | |
| No edema | 0 |
| Very mild edema (barely recognizable) | 1 |
| Mild edema (clear edge can be recognized by clear swelling) | 2 |
| Moderate edema (swelling of about 1 mm) | 3 |
| Severe edema (swelling of 1 mm or greater and spread beyond range of exposure) | 4 |

### 15.2. Observation of general condition

Local and systemic toxicological symptoms other than skin reaction were observed at the same frequency as the skin reaction observation.

### 15.3. Body weight measurement

The body weight was measured on the administration date (before administration) and date of completion of observation for all tested animals.

### 15.4. Evaluation of skin reaction

Test substances and comparative control substances were classified into the GHS [2] hazard category based on the grading results in accordance with the determination criteria.

### [Chemical Formula 19]

### Skin corrosiveness category and subcategories

| Category | Determination criteria |
|---|---|
| Corrosiveness (category 1) | Destruction of skin tissue, i.e., visible necrosis reaching the dermis through the epidermis is exhibited in at least one test animal from exposure of four hours or less. |
| Subcategory 1A | A corrosive reaction is exhibited from observation within one hour in at least one animal after exposure of three minutes or less. |
| Subcategory 1B | A corrosive reaction is exhibited from observation within 14 days in at least one animal from exposure exceeding three minutes and within one hour. |
| Subcategory 1C | A corrosive reaction is exhibited from observation within 14 days in at least one animal from exposure exceeding one hour and within four hours. |

### Skin irritability category

| Category | Determination criteria |
|---|---|
| Irritability (category 2) | (1) the mean score of erythema/eschar or edema is ≥ 2.3 and ≤ 4.0 in results of evaluation at 24, 48, and 72 hours after removal of patch or evaluation of 3 consecutive days after a skin reaction incident when the reaction is late-onset, in at least 2 out of 3 test animals, or (2) inflammation generally remains up to the end of the observation period of 14 days in at least two animals; in particular, hair loss (within a limited area), hyperkeratosis, hyperplasia, and exfoliation are considered, or (3) in some cases, there is a significant difference in reaction among animals, and a very definitive positive action is observed from chemical exposure in one animal, but not to the extent of the determination criteria specified above. |
| Mild irritability (category 3) | the mean score of erythema/eschar or edema is ≥ 1.5 and < 2.3 in results of evaluation at 24, 48, and 72 hours after removal of patch or evaluation of 3 consecutive days after a skin reaction incident when the reaction is late-onset, in at least 2 out of 3 test animals (if not classified into the irritability category specified above) |

Cases that are not classified into categories 1 to 3 are deemed not falling under the categories.

### 16. Statistical analysis

A test using statistical methodologies was not conducted.

### 17. Test results

### 17.1. Observation of skin reaction

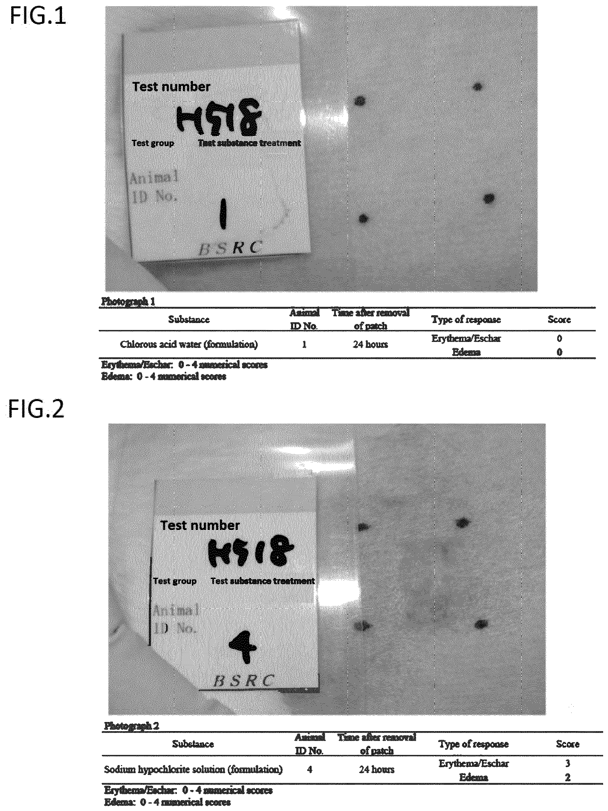

### 17.1.1. Test substance treatment group (Appendix 1-1, Photograph 1 (Figure 1))

During the observation period, a skin reaction was not observed at the sites of administration of any animals. 17.1.2. Comparative control substance treatment group (Appendix 1-2, Photograph 2 (Figure **2**))

Grade 1 erythema and grade 1 or 2 edema were found at a site of administration in all three cases from observation at 1 hour after patch removal. These reactions were subsequently exacerbated so that grade 3 erythema and edema were exhibited at 72 hours after patch removal. While edema exhibited a trend of recovery 5 days after patch removal, exacerbation of erythema to grade 4 was found 5 or 7 days after patch removal in two cases. Grade 2 or 3 erythema was observed 14 days after patch removal in all cases. Exfoliation was observed 5 or 6 days after patch removal, and scar was found on day 9 in one case. At 14 days after patch removal, a scar was found in one case, and exfoliation was found in two cases.

### 17.2. Observation of general condition (Appendix 2)

An abnormality was not found in any animal during the observation period.

### 17.3. Body weight measurement (Appendix 3)

A change in body weight was within the normal range in all animals.

### 17.4. Evaluation of skin reaction

### 17.4.1. Test substance treatment group (Table 1-1)

As a result of computing the mean score for erythema/eschar and edema of each animal at 24, 48, and 72 hours after patch removal, the scores were 0.0 in both categories in each case.

As for the GHS hazard category (skin corrosion and skin irritability), a reaction of the skin was not exhibited during the observation period, and the calculated mean score did not reach the lower limit value of any of the categories. Thus, this was classified as not falling under the categories.

### 17.4.2. Comparative control substance treatment group (Table 1-2)

As a result of computing the mean score for erythema/eschar and edema of each animal at 48 hours, 72 hours, and 4 days after patch removal when the total grade of skin reaction would be at the maximum, erythema/eschar was 3.0 in all three cases, and edema was 2.7 to 3.0.

As for the GHS hazard category (skin corrosion and skin irritability), a reaction of the skin (irritability) did not recover in all cases during the observation period, and a scar that did not recover in 14 days after patch removal determined as a corrosive reaction was observed in one case. Thus, this was classified as category 1 (corrosive).

### 18. Discussion and conclusion

The skin of rabbits was exposed to 0.5 mL of chlorous acid aqueous solution formulation or sodium hypochlorite formulation for 4 hours to study the skin irritability and corrosiveness.

As a result, a skin reaction was not observed at the site of administration of any animals during the observation period with a chlorous acid aqueous solution formulation. As for the GHS hazard category (skin corrosion and skin irritability), a reaction of the skin was not exhibited during the observation period, and the calculated mean score did not reach the lower limit value of any of the categories. Thus, this was classified as not falling under the categories.

Meanwhile, grade 1 erythema and grade 1 or 2 edema were found at a site of administration in all three cases from observation at 1 hour after patch removal with a sodium hypochlorite formulation. These reactions were subsequently exacerbated so that grade 3 erythema and edema were exhibited at 72 hours after patch removal. While edema exhibited a trend of recovery 5 days after patch removal, exacerbation of erythema to grade 4 was found 5 or 7 days after patch removal in two cases. Grade 2 or 3 erythema was observed 14 days after patch removal (final observation date) in all cases. Exfoliation was observed 5 or 6 days after patch removal, and scar was found on day 9 in one case. At 14 days after patch removal, a scar was found in one case, and exfoliation was found in two cases. As for the GHS hazard category (skin corrosion and skin irritability), a reaction of the skin (irritability) did not recover in all cases during the observation period, and a scar that did not recover in 14 days after patch removal determined as a corrosive reaction was observed in one case. Thus, this was classified as category 1 (corrosive). While exposure of 4 hours or less was not conducted, the subcategory in category 1 was predicted to be 1B or 1C.

It was determined in view of the above results that a chlorous acid aqueous solution formulation does not have a corrosive and irritable action on rabbit skin under the test conditions (GHS classification: not falling under the categories). Meanwhile, it was determined that a sodium hypochlorite formulation has a corrosive action on rabbit skin (GHS classification: category 1).

### 19. References

[1] Draize JH. Dermal toxicity. In: Appraisal of the safety of chemicals in foods, drugs and cosmetics, Association of food and drug officials of the United States; 1959: p.p. 46-53.
[2] United Nations. Globally harmonized system of classification and labelling of chemicals (GHS). Sixth revised edition, 2015.

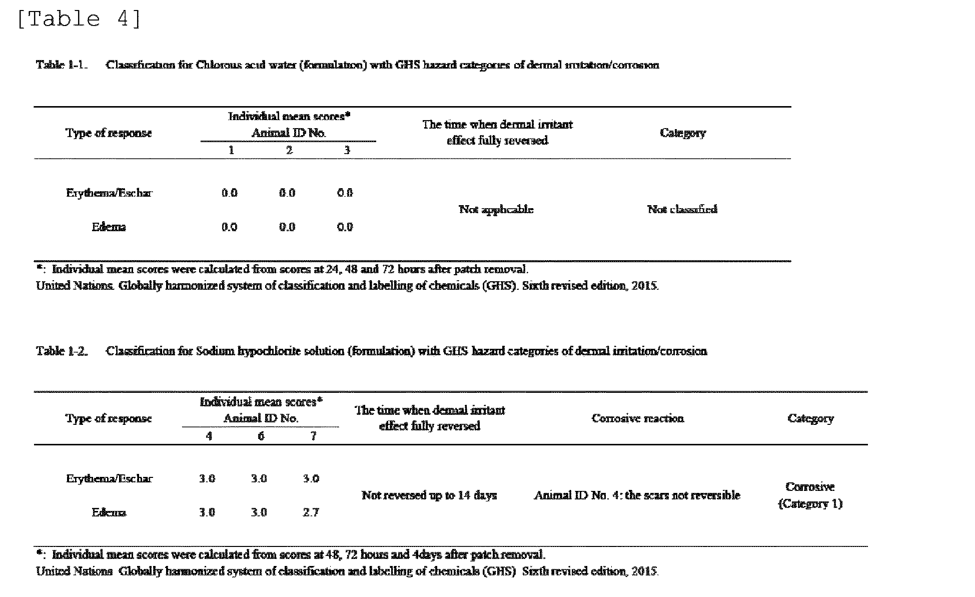

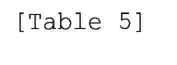

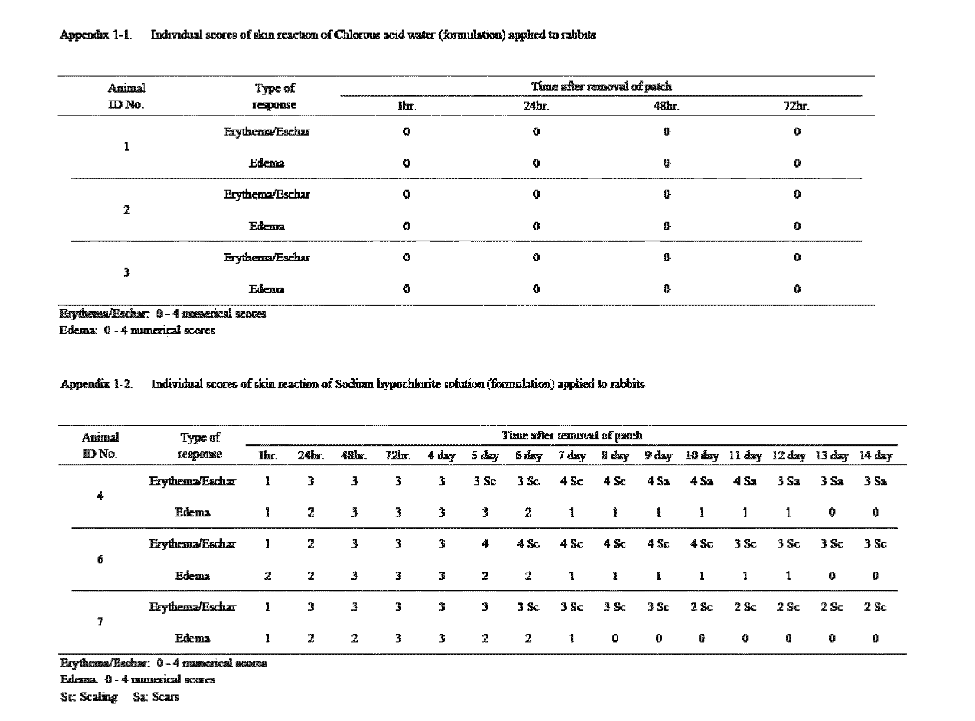

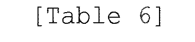

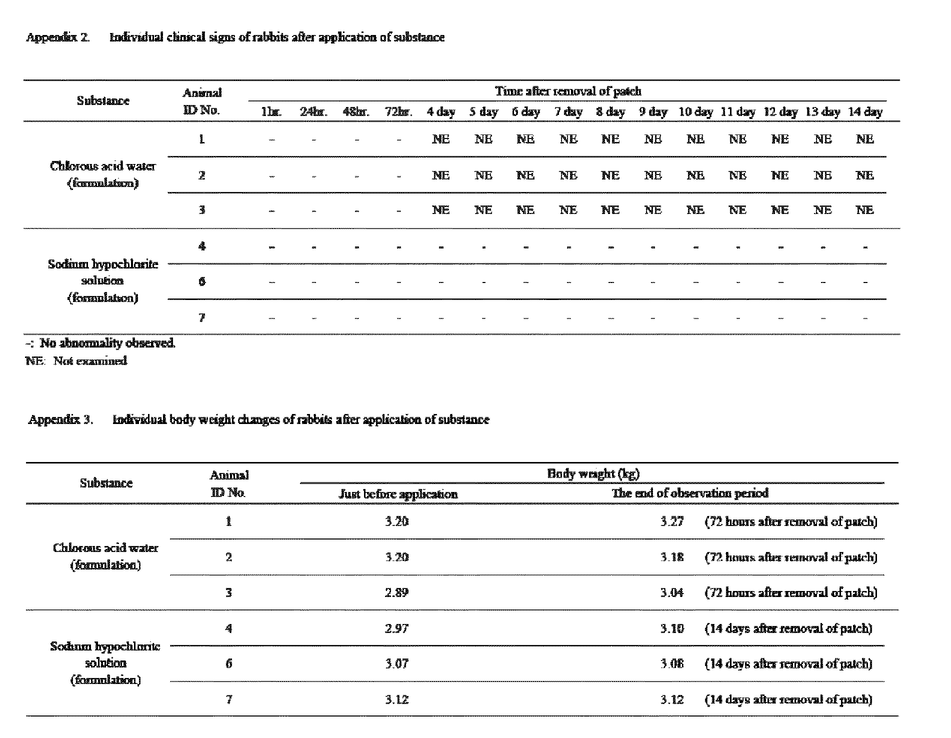

### (Example 3)

### (Eye irritability test on chlorous acid aqueous solution formulation using rabbits)

To test eye irritability and severe injury due to a chlorous acid aqueous solution formulation, an eye irritability test was conducted using three female Kbl:NZW rabbits. 0.1 mL of the test substance was administered into the conjunctival sac of the right eye of animals. The reaction of the eye to which the substance was administered was observed at 1, 24, 48, and 72 hours after administration, and the general condition was observed. In addition, the body weight was measured before administration and as of the completion of observation. At the same time as the observation of the reaction of the eye at 24 hours after administration, the corneal epithelium was stained with an aqueous sodium fluorescein solution to examine the condition thereof (presence/absence of injured site).

The results are the following.

Grade 1 redness of the conjunctiva was found in 2 out of 3 cases from observation at 1 hour after administration. The observed redness of the conjunctiva exhibited a recovery at 24 hours after patch removal. A reaction (irritable reaction) was not observed in the eye administered with the formulation in any of the animals thereafter. A stained region was not found in any of the animals from checking for an injured (stained) site on the corneal epithelium using an aqueous sodium fluorescein solution.

An abnormality in the general condition was not found, and the change in body weight was also within the normal range in all 3 rabbits during the observation period.

As for the GHS hazard category (irreversible and reversible effect on eye), a reaction indicating a severe injury was not observed during the observation period. The observed eye (irritable) reaction recovered by 72 hours after administration. Since the calculated mean score did not reach the lower limit value of any of the categories, this was classified as not falling under the categories.

It was determined in view of the above results that a chlorous acid aqueous solution formulation does not have a severe injurious and irritable action on rabbit eyes under the test conditions (GHS classification: not falling under the categories).

### 1. Title

Eye irritability test of chlorous acid aqueous solution formulation using rabbits

### 2. Test objective

To study the eye irritability and severe injury due to a chlorous acid aqueous solution formulation, an eye irritability test is conducted using rabbits.

### 3. GLP complied

-"Ministerial Ordinance Concerning the Standards for the Conduct of Non-clinical Studies on the Safety of Drugs" (ordinance of the Ministry of Health and Welfare No. 21, dated March 26, 1997)"

### 4. Referenced guideline

- OECD Guideline for Testing of Chemicals 405 (2 October 2012: Acute Eye Irritation/Corrosion)

### 5. Complied animal experiment related regulations

This test was conducted in compliance with "Act on Welfare and Management of Animals" (October 1, 1973, Act No. 105, final revision: May 30, 2014, Act No. 46) and "Standards Relating to the Care and Management of Laboratory Animals and Relief of Pain" (Notice No. 88 of the Ministry of Environment dated April 28, 2006, final revision: Notice No. 84 of the Ministry of Environment dated August 30, 2013). The test was examined and approved prior to the start of the test by the animal experiment committee of the BioSafety Research Center (approval no. 17-0031A) and conducted in accordance with the animal ethics evaluation criteria specified in "Guidelines for animal experiments" (June 2, 2014) of the BioSafety Research Center.

### 13. Test substance

### 13.1. Name

Chlorous acid water (formulation)

### 13.5. Component content

### 13.5.1. Active ingredient

0.8% chlorous acid

### 13.5.1.1. Active ingredient (actual measurement value)

Chlorous acid aqueous solution (total chlorine content:
HClO₂ = 68.46) [%] 0.84
Free chlorine (HClO₂ = 68.46) [mg/L] 448

### 13.5.2. Other components

Dipotassium hydrogen phosphate
Potassium dihydrogen phosphate

### 13.6. Stability

Stable under designated conditions until the expiration date

### 13.7. Condition of substance

### 13.7.1. Outer appearance

Bright yellow to light greenish yellow and clear liquid 13.7.2. Odor
Chlorine odor

### 13.8. Expiration date

February 7, 2018 (one year after manufacture)

### 13.9. Storage condition

Refrigerated away from light and sealed

### 13.9.1. Actual measurement value of storage temperature and storage period

- 3.1 to 7.5°C
- May 2, 2017 to June 13, 2017 (test substance: date of receipt to date of use)

### 13.10. Precaution for use

Wear a mask/glove

### 13.11. Treatment of residual test substance Disposed in entirety

### 14. Test material and method

### 14.1. Tested animal

### 14.1.1. Species

Rabbit

### 14.1.2. Line [grade]

New Zealand white species (Kbl:NZW) [SPF]

### 14.1.3. Producer

Kitayama Labes Co., Ltd.

### 14.1.4. Distributor

Oriental Yeast Co., Ltd.

### 14.1.5. Reason for selecting the test system

As the animal, white rabbits recommended by the guidelines were selected. As the line, New Zealand white species that are commonly used in this type of test were used.

### 14.1.6. Age

10-week old animals were purchased, and a test substance was administered at 11 weeks old (body weight range: 2.10 to 2.30 kg).

### 14.1.7. Number of animals purchased

4 females

### 14.1.8. Number of animals used

3 females

### 14.2. Rearing management

### 14.2.1. Rearing environment

Animals were reared in rearing room No. 7-207 [positive pressure] (W 4.8 × D 10.3 × H 2.6 m), and the reference values for controlling the environment were the following. Temperature: 17 to 23°C (actual measurement value: 20.9 to 21.4°C)
Humidity: 35 to 70% RH (actual measurement value: 50 to 67% RH)
Number of air ventilations: 8 or more/h
Lighting: 12 hours (turn on at 7:00, turn off at 19:00)

One animal was housed in each rearing cage (W 62.2 × D 75.6 × H 46.2 cm). A feeder was exchanged at a frequency of once a week.

### 14.2.2. Feed

Solid feed (RC4, Lot No. 161111, Oriental Yeast Co., Ltd.) was made freely available from a feeder.

Test results (AR-16-JP-114796-01-JA) associated with contaminants in the feed used were obtained from the manufacturer to confirm that the value was within the proposed permissible reference value of the Japan Experimental Animal Food Association.

### 14.2.3. Water supply

Tap water was made freely available from an automatic water supplying nozzle.

Water quality testing based on the Waterworks Act was commissioned to an external agency in April 2017. The test results were confirmed to be within the reference value for waterworks quality standard (Result No. K17-0031). In addition to the water quality test described above, bacteria test (general bacteria and *E. coli* test) was conducted at the BioSafety Research Center to confirm that bacteria is not detected in May, June, and July 2017 (No. GT17-05, No. GT17-06, No. GT17-07).

### 14.3. Quarantine/acclimation

The quarantine period was from delivery of the animals to measurement of body weight on the day before administration, and the acclimation period was up to before administration.

During this period, the general condition was observed once a day (excluding Saturdays and Sundays), and the body weight was measured upon delivery and day before administration (before examining the corneal epithelium).

### 14.4. Individual identification

The serial number described on the auricle of animals by Kitayama Labes Co., Ltd. was used as the animal number (Animal ID No.), and the animal ID card explicitly describing the animal number was appended to the cage to identify the animals.

### 14.5. Selection of animals

Both eyes of all animals were checked to make sure that there is no injury to the cornea by using a 2% aqueous solution (medium: injection water, Lot No. K6D73, Otsuka Pharmaceutical Factory) of sodium fluorescein (Lot No. PDK5648, Wako Pure Chemical Industries) about 24 hours before administration. On the day of administration, each animal was confirmed to have no abnormality in the general condition and change in body weight during the quarantine/acclimation period. Since redness in the conjunctiva was found at an eye of one animal expected to be administered (animal number 3), the animal was excluded from the test (surplus animal), and the remaining 3 animals were selected as the test animals.

### 14.6. Handling of surplus animals and tested animals after completion of observation

Surplus animals were transferred to the animal management department.

Tested animals were euthanized by pentobarbital sodium anesthesia.

### 14.7. Constitution of test group and reason for selecting test method

### 14.7.1. Constitution of test group

### [Chemical Formula 20]

| Test group | Number of animals | Animal number |
|---|---|---|
| Test substance treatment group | 3 | 1, 2, 4 |

### 14.7.2. Reason for selecting test method

In view of the information of a chlorous acid aqueous solution lacking irritability on the eye, a method of administering a test substance to a total of three rabbits was selected in accordance with the test guidelines.

### 14.8. Preparation of test substance

The provided test substance was directly used for administration.

### 14.9. Administration method

For administration, 0.1 mL of the undiluted test substance solution was administered to the conjunctival sac of one eye of an animal by using a MICROMAN (model M100, Gilson). To prevent loss of the test substance, the top and bottom eyelids were slowly closed and maintained for about 1 second. The eye to which the test substance was not administered was used as the untreated control eye.

### 15. Observation, measurement, and testing

### 15.1. Observation of eye reaction

The reaction in the corneal, iris, and conjunctiva in the eye administered with the substance were observed 1, 24, 48, and 72 hours after administration, and scored in accordance with the determination criteria for eye reactions (Draize [1], 1959) . For the condition of corneal epithelium, an aqueous 2% sodium fluorescein solution was administered as an eye drop 24 hours after administration, and the epithelium was washed with saline to observe the stained (injured) area of the cornea. Eyes administered with the substance for all cases were captured as reference data using a digital camera upon observation 24 hours after administration.

### Determination criteria for eye reaction

| |
|---|
| Degree of turbidity of cornea A (evaluation of the most affected region) |
| Clear with no turbidity-----------------------------------0 |
| Scattered and spread turbidity, but iris is clearly found-1 |
| Semi-transparent and readily recognizable, and iris is |
| somewhat unclear------------------------------------------2 |
| Cloudy, details of the iris cannot be observed, and the size of the pupil is barely recognized-----3 |
| Opaque, and the iris cannot be found----------------------4 [0184] |
| B Corresponding area of turbid portion in the cornea |
| 0 < to 1/4------------------------------------------------1 |
| 1/4 to 1/2------------------------------------------------2 |
| 1/2 to 3/4------------------------------------------------3 |
| 3/4 to 4/4------------------------------------------------4 |

### Iris A

| |
|---|
| Normal----------------------------------------------------0 |
| Fold, redness, swelling, or redness around the cornea beyond normal (one or combination thereof), and at least some reaction to light---------------------------1 |
| (One of) no reaction to light, bleeding, or significant tissue disorganization------------------------------------2 |
| [0186] |
| Conjunctiva A Redness of conjunctiva (palpebral conjunctiva and bulbar conjunctiva excluding the cornea and iris) |
| Blood vessels are normal----------------------------------0 |
| Clearly more redness in blood vessel than normal----------1 Widespread, dark red color, and individual blood vessels are hardly distinguishable----2 |
| Widespread beef-like redness------------------------------3 |
| [0187] |
| B Edema of the conjunctiva |
| No swelling ----------------------------------------------- 0 |
| Swelling beyond normal (including the nictitating membrane)----------1 |
| Clear swelling, and eyelid is slightly ectropion----------2 |
| Swelling resulting in eyelids to be half closed-----------3 |
| Swelling resulting in eyelids to be more than half closed-4 |

### C Secretion

| |
|---|
| No secretion is found-------------------------------------0 |
| Slightly more than normal (excluding a small amount observed in the medial angle of the eye of a normal animal)--------1 |
| Secretion that moisturizes the eyelid and hair in the vicinity thereof------------------------------------------2 |
| Secretion to the extent that most of the eyelid, hair, and the surrounding area is moist-----------------------------3 |

### 15.2. Observation of general condition

Local and systemic toxicological symptoms other than eye reactions were observed at the same frequency as the eye reaction observation.

### 15.3. Body weight measurement

The body weight was measured on the administration date (before administration) and date of completion of observation for all test animals.

### 15.4. Evaluation of eye reaction

Substances were classified into GHS [2] hazard categories based on the grading results in accordance with the determination criteria.

### [Chemical Formula 21]

### Categories for severe injury to the eye/irreversible action on eye

| Category | Determination criteria |
|---|---|
| Category 1 | Substances exhibiting the following action: |
| Severe injury to the eye/irreversible action on eye | (a) An action that is expected to be reversible to the corneal, iris, or conjunctiva is found on at least one animal. An action that is not completely reversed is found during the observation period that is generally 21 days; and/or |
| | (b) a positive reaction is obtained, where the |
| | calculated mean score of evaluation at 24, 48, and 72 hours after administering the test substance as an eye drop is (i) corneal turbidity ≥ 3 and/or (ii) iritis > 1.5, and in at least 2 out of 3 test animals. |

### Categories for reversible action on eye

| Category | Determination criteria |
|---|---|
| Category 2 | Substance with a possibility of reversible eye irritating action |
| Category 2A | The following reaction is obtained in at least 2 out of 3 test animals. |
| | The calculated mean score of evaluation at 24, 48, and 72 hours after administering the test substance as an eye drop is |
| | (a) corneal turbidity ≥ 1 and/or |
| | (b) iritis ≥ 1 and/or |
| | (c) redness in the conjunctiva ≥ 2 and/or |
| | (d) conjunctival edema ≥ 2 and complete recovery within the observation period that is generally 21 days. |
| Category 2B | If the aforementioned action is completely reversible within the 7 day observation period in category 2A, eye irritability is deemed a mild eye irritability (category 2B). |

Cases that are not classified into category 1 or 2 are deemed as not falling under the categories.

### 16. Statistical analysis

A test using statistical methodologies was not conducted.

### 17. Test results

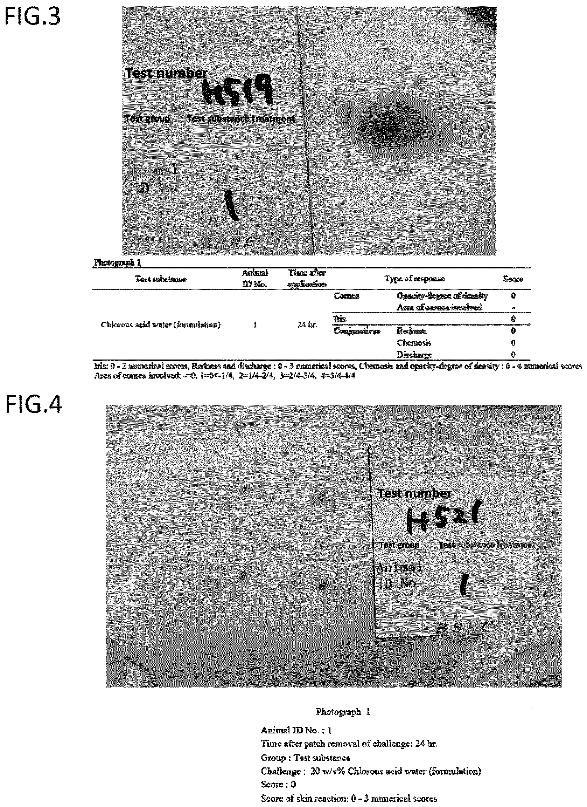

### 17.1. Observation of eye reaction (Appendix 1 and 2, Photograph 1 (Figure 3))

Grade 1 redness in the conjunctiva was found in 2 out of 3 cases from observation at 1 hour after administration. The observed redness in the conjunctiva recovered at 24 hours after patch removal. An eye reaction (irritable reaction) was not observed thereafter in an eye administered with a substance in any animal. A stained area was not found in any animal when examining for injured (stained) sites in the corneal epithelium using an aqueous sodium fluorescein solution.

### 17.2. Observation of general condition (Appendix 3)

An abnormality was not found in any animal during the observation period.

### 17.3. Body weight measurement (Appendix 4)

A change in body weight was within the normal range in all animals.

### 17.4. Evaluation of eye reaction (Table 1)

As a result of computing the mean score for the cornea, iris, and conjunctival redness and edema of each animal at 24, 48, and 72 after administration, the scores for the cornea, iris, and conjunctival redness and edema were 0.0 in all three case.

As for the GHS hazard category (irreversible and reversible effect on eye), a reaction exhibiting severe injury was not found during the observation period, and the observed eye (irritating) reaction recovered by 72 hours after administration, and since the calculated mean score did not reach the lower limit value of any of the categories, this was classified as not falling under the categories.

### 18. Discussion and conclusion

0.1 mL of chlorous acid aqueous solution formulation was administered to an eye of a rabbit to study the irritability and severe injury to the eye.

As a result, grade 1 redness in the conjunctiva was found in 2 out of 3 cases from observation at 1 hour after administration. The observed redness in the conjunctiva recovered at 24 hours after patch removal. An eye reaction (irritable reaction) was not observed thereafter in an eye administered with a substance in any animal. A stained area was not found in any animal when examining for injured (stained) sites in the corneal epithelium using an aqueous sodium fluorescein solution.

As for the GHS hazard category (irreversible and reversible effect on eye), a reaction exhibiting severe injury was not found during the observation period, and the observed eye (irritating) reaction recovered by 72 hours after administration, and since the calculated mean score did not reach the lower limit value of any of the categories, this was classified as not falling under the categories.

It was determined in view of the above results that a chlorous acid aqueous solution formulation does not have a severe injury causing action or irritating action on rabbit eyes under the test conditions (GHS classification: not falling under the categories).

### 19. References

[1] Draize JH. Dermal toxicity. In: Appraisal of the safety of chemicals in foods, drugs and cosmetics, Association of food and drug officials of the United States; 1959: p.p. 46-53.
[2] United Nations. Globally harmonized system of classification and labelling of chemicals (GHS). Sixth revised edition, 2015.

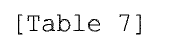

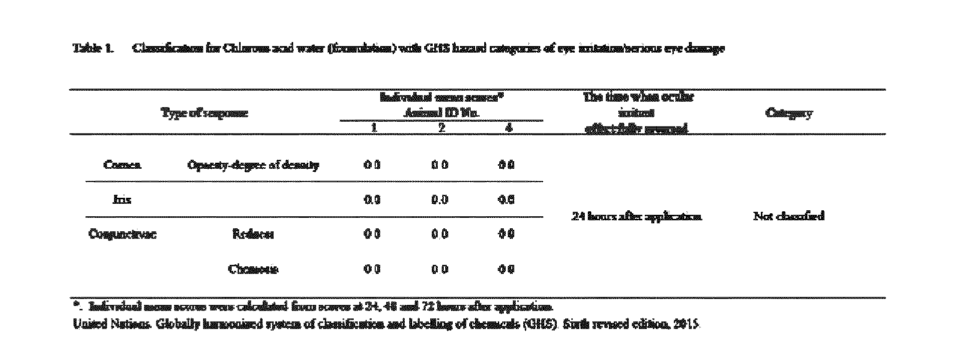

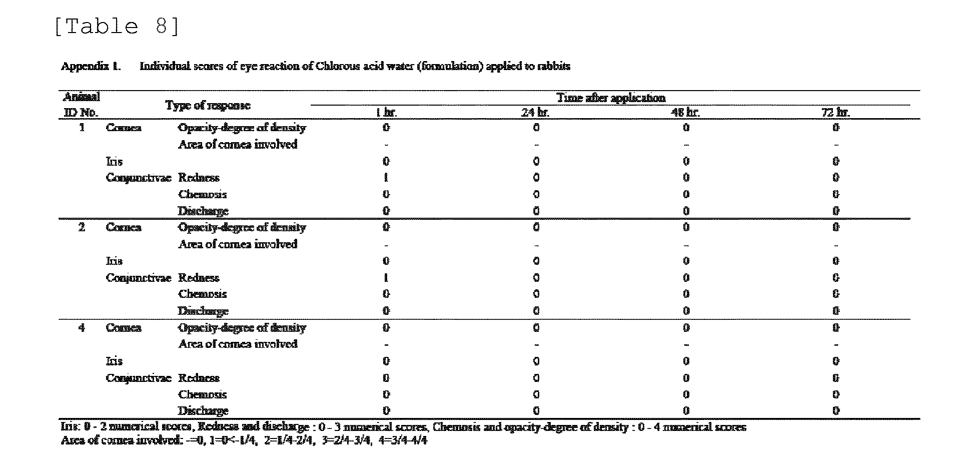

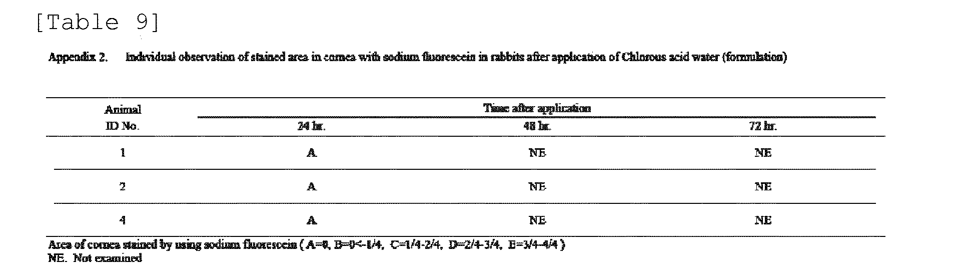

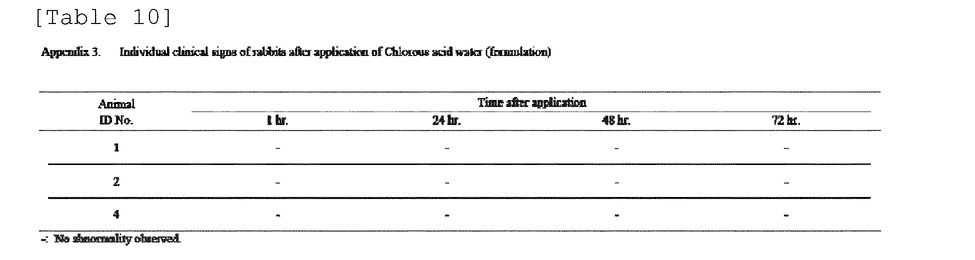

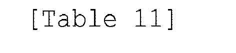

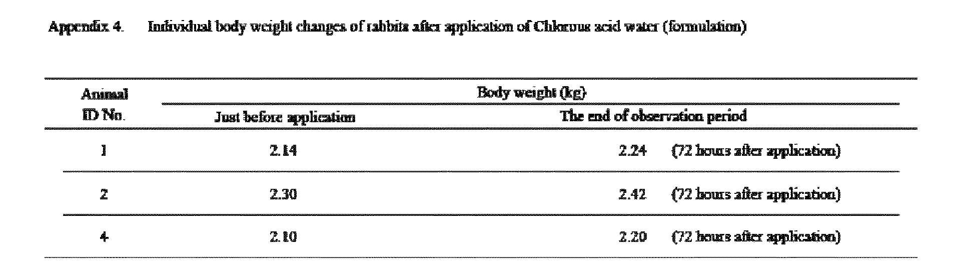

### (Example 4)

### (Skin sensitization test on chlorous acid aqueous solution formulation using guinea pigs (Maximization Test))

To find information related to the skin sensitization of a chlorous acid aqueous solution formulation, a Maximization Test was conducted using 20 female Slc:Hartley guinea pigs (SPF). The medium and treatment concentration in the skin sensitization test were determined based on the results of a preliminary test conducted in advance. In the test substance treatment group, 0.2 w/v% test substance solution (medium: distilled water) was intradermally injected in the first sensitization treatment using 10 animals, and a closed patch of 100% test substance solution (undiluted solution) was applied for 48 hours in the second sensitization treatment. A closed patch of 20 w/v% test substance solution was applied for 24 hours for elicitation treatment. 5 guinea pigs were designated as a negative control group using a medium in intradermal sensitization treatment, and 5 guinea pigs were designated as a positive control group using a positive control substance α-Hexylcinnamaldehyde (HCA) as control groups. A skin reaction was observed for all sites of elicitation treatment of each group.

The results are the following.

A reaction of the skin was not observed for any of the animals during the observation of the site treated with a test substance for elicitation. The skin sensitization rate of the test substance was calculated to be 0%, which did not reach the lower limit value of any of the GHS hazard categories (skin sensitization). Thus, this was classified as not falling under the categories. Meanwhile, during the observation of the site treated with a positive control substance for elicitation, a grade 3 skin reaction was observed in all 5 cases of the positive control group, while none of the animals exhibited a skin reaction in the negative control group. The skin sensitization rate of a positive control substance was calculated as 100%, so that skin sensitization of the positive control substance HCA was confirmed. Thus, it was determined that skin sensitization of a chlorous acid aqueous solution formulation was property evaluated in the test.

It was determined in view of the above results that a chlorous acid aqueous solution formulation is not a skin sensitizing substance under the test conditions (GHS classification: not falling under the categories).

### 1. Title

Skin sensitization test on chlorous acid aqueous solution formulation using guinea pigs (Maximization Test)

### 2. Test objective

The objective is to conduct a skin sensitization test through Maximization by using guinea pigs to obtain information regarding skin sensitization of a chlorous acid aqueous solution formulation.

### 3. GLP complied

-"Ministerial Ordinance Concerning the Standards for the Conduct of Non-clinical Studies on the Safety of Drugs" (ordinance of the Ministry of Health and Welfare No. 21, dated March 26, 1997)"

### 4. Referenced guideline

- Guideline for Toxicological test required for application for approval of manufacture (import) of pharmaceutical products (September 11, 1989, Pharmaceutical Affairs Council, I, No. 24)
- OECD Guideline for Testing of Chemicals 406 (17th July 1992: Skin Sensitization)

### 5. Complied animal experiment related regulations

This test was conducted in compliance with "Act on Welfare and Management of Animals" (October 1, 1973, Act No. 105, final revision: May 30, 2014, Act No. 46) and "Standards Relating to the Care and Management of Laboratory Animals and Relief of Pain" (Notice No. 88 of the Ministry of Environment dated April 28, 2006, final revision: Notice No. 84 of the Ministry of Environment dated August 30, 2013). The test was examined and approved prior to the start of the test by the animal experiment committee of the BioSafety Research Center (approval no.

17-0038A) and conducted in accordance with the animal ethics evaluation criteria specified in "Guidelines for animal experiments" (June 2, 2014) of the BioSafety Research Center.

### [Definition of Day]

The day of starting sensitization treatment is defined as day 0, and the day before is defined as day -1.

### 13. Test substance and control substance

### 13.1. Test substance

### 13.1.1 Name

Chlorous acid water (formulation)

### 13.1.5 Component content

### 13.1.5.1. Active ingredient

0.8% chlorous acid

### 13.1.5.1.1. Active ingredient (actual measurement value) Chlorous acid aqueous solution (total chlorine content: HClO₂ = 68.46) [%] 0.84

Free chlorine (HClO₂ = 68.46) [mg/L] 448

### 13.1.5.2. Other components

Dipotassium hydrogen phosphate
Potassium dihydrogen phosphate

### 13.1.6. Stability

Stable under designated conditions until the expiration date

### 13.1.7. Condition of substance

### 13.1.7.1. Outer appearance

Bright yellow to light greenish yellow and clear liquid 13.1.7.2. Odor
Chlorine odor

### 13.1.8. Expiration date

February 7, 2018 (one year after manufacture)

### 13.1.9. Storage condition

Refrigerated away from light and sealed

### 13.1.9.1. Actual measurement value of storage temperature and storage period

- 3.1 to 7.5°C
- May 2, 2017 to July 11, 2017 (test substance: date of receipt to date of final use)

### 13.1.10. Precaution for use

Wear a mask/glove

### 13.1.11. Treatment of residual test substance Returned to manager of test substance, etc. and disposed

### 13.2. Positive control substance

α-Hexylcinnamaldehyde (HCA), which is widely used in sensitization tests and exemplified in the guidelines, was selected as a positive control substance.

### 13.2.1. Name

α-Hexylcinnamaldehyde

### 13.2.2. Abbreviation

HCA

### 13.2.3. Manufacturer

Wako Pure Chemical Industries

### 13.2.4. Lot number

SAF6701

### 13.2.5. CAS No.

101-86-0

### 13.2.6. Condition of substance

Liquid

### 13.2.7. Storage condition

Room temperature (tolerable temperature: 1 to 30°C)

### 13.2.8. Precaution for use

Wear a mask/glove

### 14. Test material and method

### 14.1. Tested animal

### 14.1.1. Species

Guinea pig

### 14.1.2. Line [grade]

Slc:Hartley [SPF]

### 14.1.3. Producer

Japan SLC, Inc.

### 14.1.4. Reason for selecting the test system

Guinea pigs recommended by the test guidelines were selected. As the line, Slc:Hartley was selected in view of the sensitivity to known chemical substances, genetic stability, etc.

### 14.1.5. Age

6-week old animals were purchased, and administration was started at 7 weeks old (body weight range: 384 to 452 g). 14.1.6. Number of animals purchased
22 females

### 14.1.7. Number of animals used

20 females

### 14.2. Rearing management

### 14.2.1. Rearing environment

Animals were reared in rearing room No. 7-201 [positive pressure] (W 6.4 × D 10.3 × H 2.6 m), and the reference values for controlling the environment were the following.
Temperature: 20 to 26°C (actual measurement value: 22.8 to 23.3°C)
Humidity: 35 to 70% RH (actual measurement value: 48 to 61% RH)
Number of air ventilations: 12 or more/h
Lighting: 12 hours (turn on at 7:00, turn off at 19:00)
One animal was housed in each aluminum front and floor stainless mesh rearing cage (W 29.1 × D 26.3 × H 18.0 cm) by using a water-washable rearing equipment (Toyo Riko). A rearing cage was exchanged at a frequency of every other week, and a feeder was exchanged at a frequency of once a week.

### 14.2.2. Feed

Solid feed (RC4, Lot No. 161111, 170404, Oriental Yeast Co., Ltd.) was made freely available to the animals from a feeder.

Analysis results (AR-16-JP-114796-01-JA, AR-17-JP-004057-01-JA) associated with contaminants in the feed were obtained from the manufacturer to confirm that the value was within the proposed permissible reference value of the Japan Experimental Animal Food Association.

### 14.2.3. Water supply

Tap water was made freely available from an automatic water supplying nozzle to the animals.

Water quality testing based on the Waterworks Act was commissioned to an external agency in April 2017. The test results were confirmed to be within the reference value for waterworks quality standard (Result No. K17-0031). In addition to the water quality test described above, bacteria test (general bacteria and E. coli test) was conducted at the BioSafety Research Center to confirm that bacteria is not detected in June, July, and August 2017 (No. GT17-06, No. GT17-07, No. GT17-08).

### 14.3. Quarantine/acclimation

The quarantine and acclimation period was from delivery of the animals to start of sensitization.

During the quarantine/acclimation period, the general condition of the animals was observed once a day (excluding Saturdays and Sundays), and the animals were acclimated to the test environment. The body weight was also measured upon delivery and before starting sensitization treatment (as of end of the quarantine/acclimation period). An abnormality was not found in any animal in the observation/measurement.

### 14.4 Grouping

The animals were grouped on day 0 (day of starting sensitization treatment).

Since an animal exhibiting an abnormality was not found during the quarantine/acclimation period, the animals were assigned to each group in order of the numbers, and the rest of the animals (animal numbers 21 and 22) were handled as surplus animals.

### 14.5. Individual identification

For identification of animals, a serial number (animal number) was described on the auricle by using a permanent marker upon delivery of the animals, and the animal identification card (ID card) explicitly describing the animal number was appended to the cage. After grouping, the animals were identified by appending a label explicitly describing the animal number and test group to the cage.

### 14.6. Handling of surplus animals and tested animals after completion of observation

Surplus animals were transferred to the animal management department.

Tested animals were euthanized by carbon dioxide inhalation.

14.7. Constitution of test group and reason for determining the concentration

### 14.7.1. Constitution of test group

### [Chemical Formula 22]

| Test group | Number of animals | First sensitization treatment (intradermal injection) | Second sensitization treatment (closed patch) | Elicitation treatment (closed patch) |
|---|---|---|---|---|
| Test substance treatment | 10 | (1) distilled water/FCA emulsion | 100% test substance solution (undiluted solution) | 20 w/v% test substance solution (medium: distilled water) |
| group | | (2) 0.2 w/v% test substance solution (medium: distilled water) | | |
| | | (3) 0.2 w/v% test substance solution/FCA emulsion | | |
| Negative control group | 5 | (1) distilled water/FCA emulsion | - | 20 w/v% test substance solution (medium: distilled water) |
| | | (2) distilled water | | |
| | | (3) distilled water/FCA emulsion | | |
| | | | | 10 w/v% HCA solution (medium: liquid paraffin) |
| Positive control group | 5 | (1) distilled water/FCA emulsion | 100% HCA solution (undiluted solution) | 10 w/v% HCA solution (medium: liquid paraffin) |
| | | (2) 10 w/v% HCA solution (medium: corn oil) | | |
| | | (3) 10 w/v% HCA/FCA emulsion | | |

| | | | | |
|---|---|---|---|---|
| FCA: Freund's complete adjuvant -: treated only with a patch | | | | |

### 14.7.2. Reason for determining the concentration

The concentration of a test substance for treatment was determined based on the results of a preliminary test (test number H520 (697-003)] (GLP non-applicable). In the preliminary test, test substance solutions of a total of 9 concentrations at 0.1, 0.2, 0.5, 1, 2, 5, 10, 20, and 50 w/v% (medium: distilled water) were intradermally injected into one animal at two sites at 0.1 mL each. As a result, a corrosive reaction (white spots and eschar formation at the administration sites) were found at the administration sites at a concentration of 0.5 w/v% or higher at 24 hours after administration. A grade 2 skin reaction was found at 0.2 w/v% administration sites, and a grade 1 or 2 skin reaction was found at 0.1 w/v% administration sites. Further, a total of three concentrations of the test substance at 4 and 20 w/v% (medium: distill water) and 100% (undiluted solution) were applied via a closed patch for 24 hours to two animals. As a result, a moderate skin reaction was found at 100% of the patched sites at 24 hours after patch removal, while a skin reaction was not found at a patched site of 20 w/v% or less. An abnormality in the general condition was not observed in animals subjected to intradermal injection or closed patch.

In view of the above results, the concentration was determined as 0.2 w/v%, which is the maximum concentration where a moderate (grade 2) skin reaction is achieved but a corrosive reaction is not exhibited, in intradermal injection for sensitization treatment in this test, and 100%, which is the maximum concentration where a moderate skin reaction was observed, in closed patch for sensitization, and 20 w/v%, which is the maximum concentration where a skin reaction was not observed, in elicitation treatment.

### 14.8. Reason for selecting the test method

Maximization Test [1] recommended by the test guidelines was selected from known skin sensitization testing methods.

### 14.9. Preparation of administered solution

The administered solution was prepared upon use, on the day of each administration. The administered solution was not analyzed.

### 14.9.1. First sensitization treatment (intradermal injection)

### 14.9.1.1. Test substance treatment group

### (1) Distilled water/FCA emulsion

Two Luer lock glass syringes were prepared. 5 mL of FCA (Lot No. 6231897, DIFCO) was aspirated into one syringe, and the same volume of distill water (injection water, Lot No. K6D73, Otsuka Pharmaceutical Factory) was aspirated into the other syringe. The two syringes were connected with a metal joint. The cylinders of the two syringes were alternatingly pushed to admix the two solutions to prepare an emulsion.

### (2) 0.2 w/v% test substance solution

0.04 g of test substance was weighed out. Distilled water (injection water, Lot No. K6D73) was added thereto and stirred to achieve a constant volume of 20 mL.

### (3) 0.2 w/v% test substance/FCA emulsion

0.04 g of test substance was weighed out. Distilled water was added thereto and stirred to achieve a constant volume of 10 mL (0.4 w/v% test substance solution). Two Luer lock glass syringes were prepared. 2 mL of 0.4 w/v% test substance solution was aspirated into one syringe, and the same volume of FCA was aspirated into the other syringe. The two syringes were then connected with a metal joint. The cylinders of the two syringes were alternatingly pushed to admix the two solutions to prepare an emulsion.

### 14.9.1.2. Negative control group

### (1) Distilled water/FCA emulsion

The substances prepared in section 14.9.1.1. were used.

### (2) Distilled water

Distilled water was used.

### (3) Distilled water/FCA emulsion

The substances prepared in section 14.9.1.1. were used.

### 14.9.1.3. Positive control group

### (1) Distilled water/FCA emulsion

The substances prepared in section 14.9.1.1. were used.

### (2) 10 w/v% HCA solution

0.3 g of HCA was weighed out. Corn oil (Lot No. SAG4916, Wako Pure Chemical Industries) was added thereto and stirred to achieve a constant volume of 3 mL.

### (3) 10 w/v% HCA/FCA emulsion

0.2 g of HCA was weighed out. FCA was added thereto and stirred to achieve a constant volume of 1 mL (20 w/v% HCA·FCA mixture). Two Luer lock glass syringes were prepared. 20 w/v% HCA·FCA mixture was aspirated into one syringe, and the same volume of distilled water was aspirated into the other syringe. The two syringes were connected with a metal joint. The cylinders of the two syringes were alternatingly pushed to admix the two solutions to prepare an emulsion.

### 14.9.2. 10% sodium dodecyl sulfate mixture

1.0 g of sodium dodecyl sulfate (Lot No. SAG9168, Wako Pure Chemical Industries) was weighed out. 9.0 g of petroleum jelly (Sun White P-1, Lot No. 16F3, NIKKO RICA) was added, and then two substances were mixed until a homogenous paste was produced using a spatula.

### 14.9.3. Second sensitization treatment (closed patch)

### 14.9.3.1. Test substance treatment group (100% test substance solution)

An undiluted solution of a test substance was used.

### 14.9.3.2. Negative control group

An administered solution was not used.

### 14.9.3.3. Positive control group (100% HCA solution)

An undiluted solution of HCA was used.

### 14.9.4. Elicitation treatment (closed patch)

### 14.9.4.1. Test substance treatment group and negative control group (20 w/v% test substance solution)

2 g of test substance was weighed out. Distilled water (injection water, Lot No. K6D73) was added thereto and stirred to achieve a constant volume of 10 mL.

### 14.9.4.2. Positive control group and negative control group (10 w/v% HCA solution)

0.3 g of HCA was weighed out. Liquid paraffin (Lot No. 816186, Yoshida Pharmaceutical) was added thereto and stirred to achieve a constant volume of 3 mL.

### 14.10. Administration method

The administration method was in accordance with the Maximization Test.

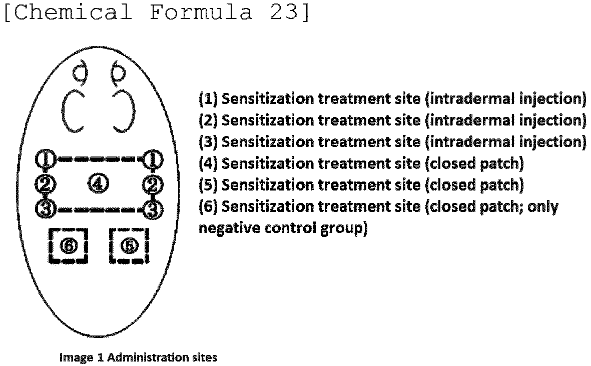

### 14.10.1. First sensitization treatment (day 0)

The chest and back of an animal were shaved using an electric clipper and shaver. A 2 × 4 cm administration section (image 1, site surrounded by (1) to (3)) was provided by marking the shaved portion with a permanent marker.

The prepared administered solutions (1) to (3) (section 14.9.1) of each test group were intradermally injected at 0.1 mL each to correspond to administration sites (1) to (3) in image 1.

### 14.10.2. Open patch of 10% sodium dodecyl sulfate mixture (day 7)

The chest and back (image 1, site (4)) of an animal were shaved using an electric clipper and shaver on day 7. Open patch of 10% sodium dodecyl sulfate mixture (section 14.9.2) was applied thinly and homogeneously at the same site to a test substance treatment group and negative control group.

### 14.10.3. Second sensitization treatment (days 8 to 10)

0.2 mL of administration solution (section 14.9.3) of each test group was homogeneously applied to 2 × 4 cm filter paper covered with a surgical tape (width: 2 inches, Blenderm^{™}, 3M) on one side by using a pipette (MICROMAN^{®}, model M1000, Gilson). This patch was applied to the administration segment (image 1, site (4)) to correspond to the site of administration of each test group, and then secured with a surgical tape (width: 2 inches, Transpore^{™}, 3M). Only filter paper is applied to a negative control group and similarly secured with a surgical tape. After 48 hours of closed patch, the patch was removed.

### 14.10.4. Elicitation treatment (days 22 to 23)

The chest and back (image 1, sites (5) and (6)) of an animal were shaved using an electric clipper and shaver. 0.1 mL of administration solution (section 14.9.4) of each test group was homogeneously applied to 2 × 2 cm filter paper covered with a surgical tape (width: 2 inches, Blenderm^{™}, 3M) on one side by using a pipette (MICROMAN^{®}, model M100, Gilson). This patch was applied to the administration segments (image 1, sites (5) and (6)) to correspond to the site of administration of each test group, and then secured with a surgical tape (width: 2 inches, Transpore^{™}, 3M). After closed patch for 24 hours, the patch was removed, and the elicitation treatment site was marked with a permanent marker. The test substance remaining on the elicitation treatment site was removed by using distilled water and tissue paper.

### 15. Observation, measurement, and testing

### 15.1. Observation of skin reaction

The elicitation treatment sites were observed 24 and 48 hours after removal of a patch in elicitation treatment, and the degree of skin reaction was scored in accordance with the following determination criteria for skin reactions. Sites of elicitation treatment in a representative example of each group were captured as reference data using a digital camera upon observation at 24 hours after patch removal.

### [Chemical Formula 24]

| **Magnusson and Kligman scale** | | |
|---|---|---|
| | **Patch test reaction** | **Grading scale** |
| No visible change | | 0 |
| Discrete or patchy erythema | | 1 |
| Moderate and confluent erythema | | 2 |
| Intense erythema and swelling | | 3 |

### 15.2. Observation of general condition

During the observation period (Day 0 to 25), the general condition including skin reactions of sensitization treatment sites was observed once a day (excluding Saturdays and Sundays).

### 15.3. Body weight measurement

The body weight was measured before starting sensitization treatment (day 0) and the day of completion of observation of elicitation treatment sites (day 25).

### 15.4. Evaluation of skin sensitization

The skin reaction expression rate was computed for each elicitation treatment site.

Among the graded scores in section 15.1., the highest score of each animal was used as the grade of the animal. An animal showing a higher grade than the highest grade found in the negative control group in the test substance treatment group was determined as a sensitization positive animal, and classified to GHS [2] hazard category based on the skin sensitization rate.

The skin sensitization rate is calculated as [(number of sensitization positive animals/number of animals used) × 100] .

Expression of a skin reaction at an elicitation treatment site was confirmed for the positive control group (skin sensitization rate of 30% or greater) to confirm that the experimental methodology was appropriate.

### Hazard category and subcategory of skin sensitization substance (GHS)

### [Chemical Formula 25]

| Category | Determination criteria |
|---|---|
| Category 1 | When a positive result is obtained from a suitable animal test, a test substance is classified as a skin sensitization substance. |
| Subcategory 1A | A significant sensitization may occur in humans from the high sensitizing ability in animals. The severity of reaction is also considered. Reaction of ≥ 30% with an intradermal dosage of ≤ 0.1%, or reaction of ≥ 60% with an intradermal dosage of > 0.1% and ≤ 1% |
| Subcategory 1B | Sensitization may occur in humans from low to moderate sensitizing ability in animals. The severity of reaction is also considered. Reaction of ≥ 30% and < 60% with an intradermal dosage of > 0.1% and ≤ 1%, or reaction of ≥ 30% with an intradermal dosage of > 1% |

When not classified in any of the categories described above, the substance is classified as not falling under the categories.

### 16. Statistical analysis

A test using statistical methodologies was not conducted.

### 17. Test results

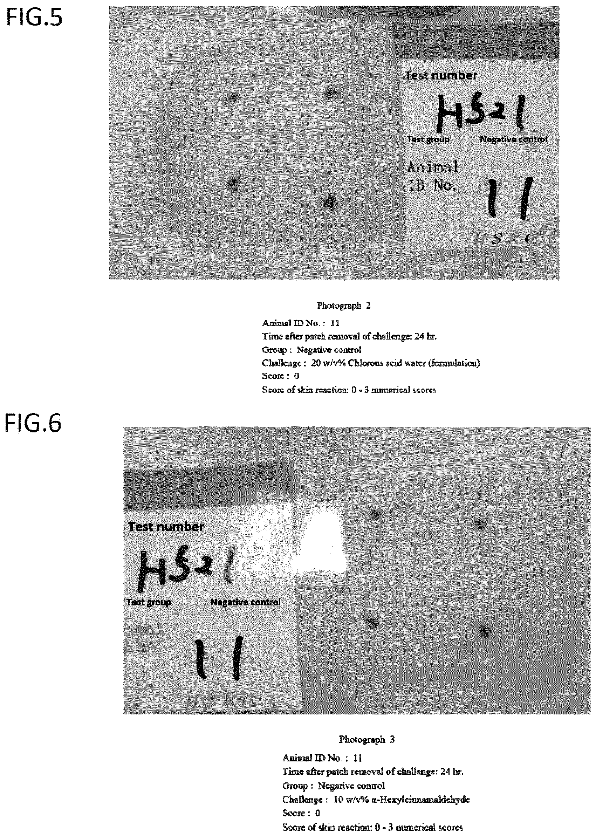

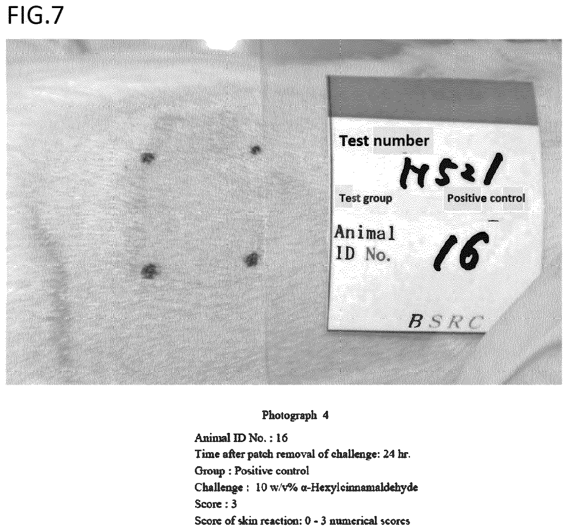

### 17.1. Observation of skin reaction (Appendix 1, Photographs 1 to 4 (Figure 4 to Figure 7))

None of the animals exhibited a skin reaction during observation of elicitation treatment sites treated with a test substance. Meanwhile, in the observation of an elicitation treatment site of the positive control substance, a grade 3 skin reaction was observed in all 5 cases in the positive control group, and none of the animals exhibited a skin reaction in the negative control group.

### 17.2. Observation of general condition (Table 2)

Erythema/edema was observed on day 1 and thereafter, and eschar formation was observed on day 4 or day 7 and thereafter at a site of intradermal injection, which is understood to be due to irritability of FCA and test substance or the positive control substance, in all animals. Further, erythema/edema was observed on day 8 or day 10 and thereafter, and some eschar formation or exfoliation was observed thereafter at a site of closed patch (second sensitization treatment) understood to be due to sodium dodecyl sulfate treatment or positive control substance treatment.

### 17.3. Body weight measurement (Appendix 2)

The body weight as of the end of observation of all animals increased relative to the start of sensitization (day 0).

### 17.4. Evaluation of skin sensitization (Table 1)

The skin sensitization rates of the test substance treatment group and the positive control group were calculated as 0% and 100%, respectively, from the result of observing skin reactions. As for the GHS hazard category (skin sensitization) of the test substance, the calculated skin sensitization rate did not reach the lower limit value of any of the categories. Thus, this was classified as not falling under the categories. Further, the skin sensitization rate was 30% or higher in the positive control group, so that skin sensitization was confirmed.

### 18. Discussion and conclusion

Maximization test was conducted using guinea pigs in order to obtain information on skin sensitization of chlorous acid aqueous solution formulations.

As a result, a skin reaction was not found in any of the animals during observation of a site of elicitation treatment, which was treated with a test substance. The skin sensitization rate was calculated to be 0% and did not reach the lower limit value of any of the GHS hazard categories (skin sensitization), so that this was classified as not falling under the categories. Meanwhile, grade 3 skin reactions were found in all five cases of the positive control group from observation of elicitation treatment sites of the positive control substance, while none of the animals exhibited a skin reaction in the negative control group. The skin sensitization rate was calculated to be 100% and the skin sensitization of HCA, which is a positive control substance, was confirmed. Thus, it was determined that skin sensitization of a chlorous acid aqueous solution formulation was properly evaluated in the test.

In addition, there was no event that affected the sensitization evaluation in observation of the general condition and measurement results of the body weight.

It was determined in view of the above results that a chlorous acid aqueous solution formulation is not a skin sensitizing substance under the test conditions (GHS classification: not falling under the categories).

### 19. References

[1] Magnusson B, Kligman AM. The identification of contact allergens by animal assay. The guinea pig maximization test. J Invest Dermatol 1969; 52: 268.
[2] United Nations. Globally harmonized system of classification and labelling of chemicals (GHS). Sixth revised edition, 2015.

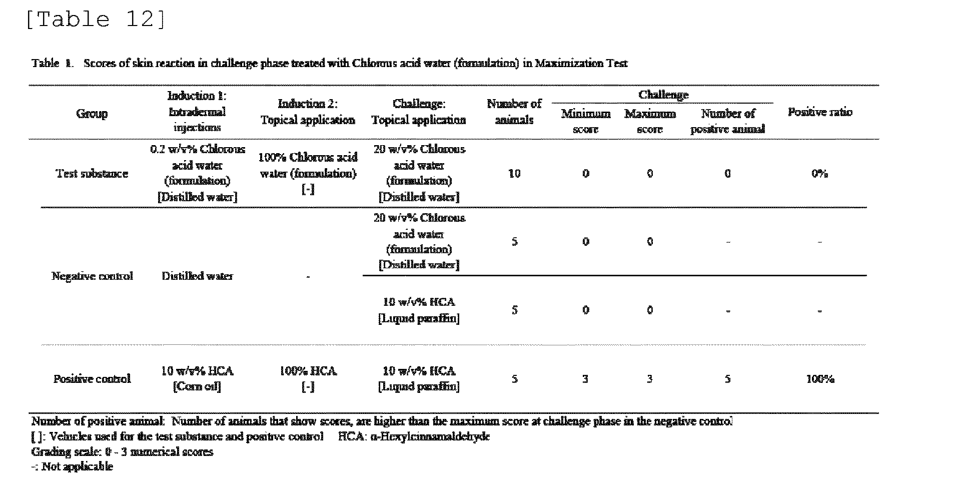

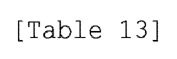

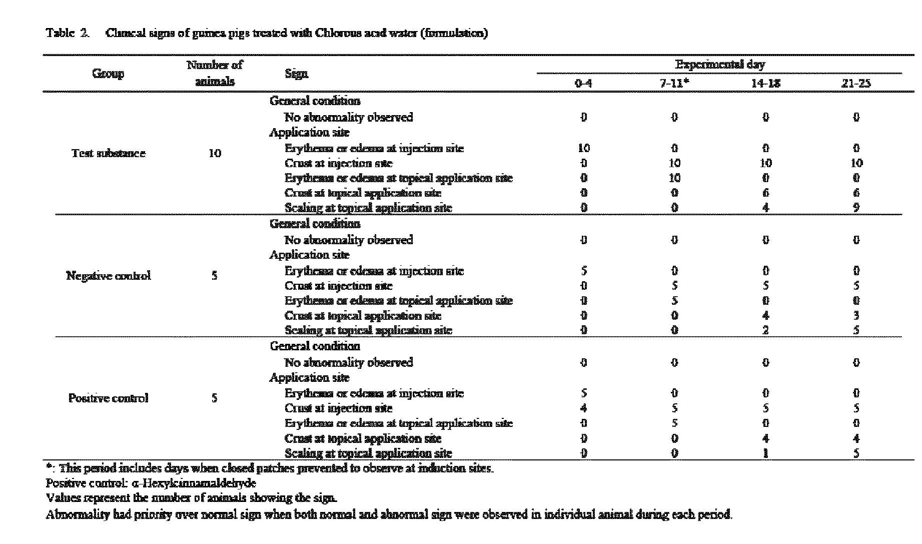

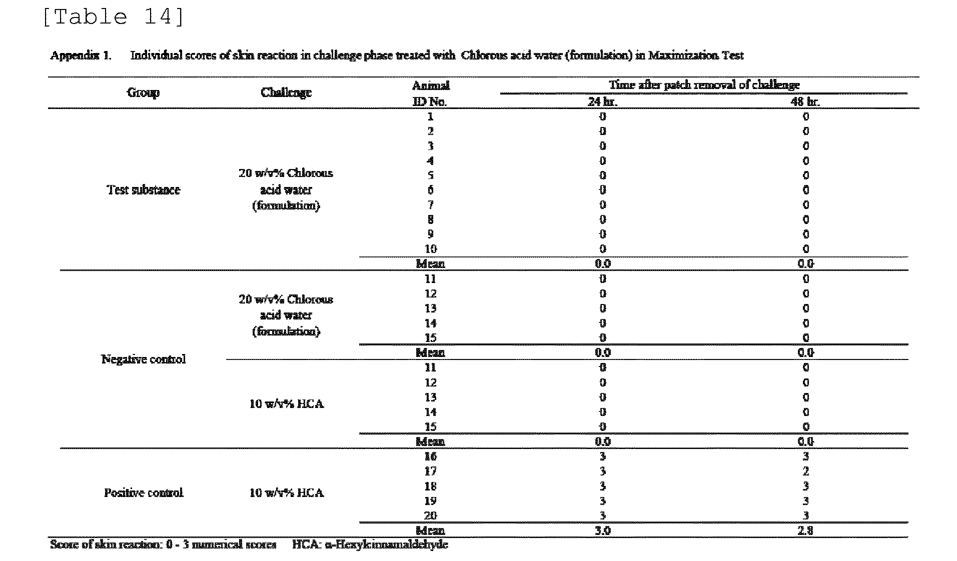

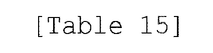

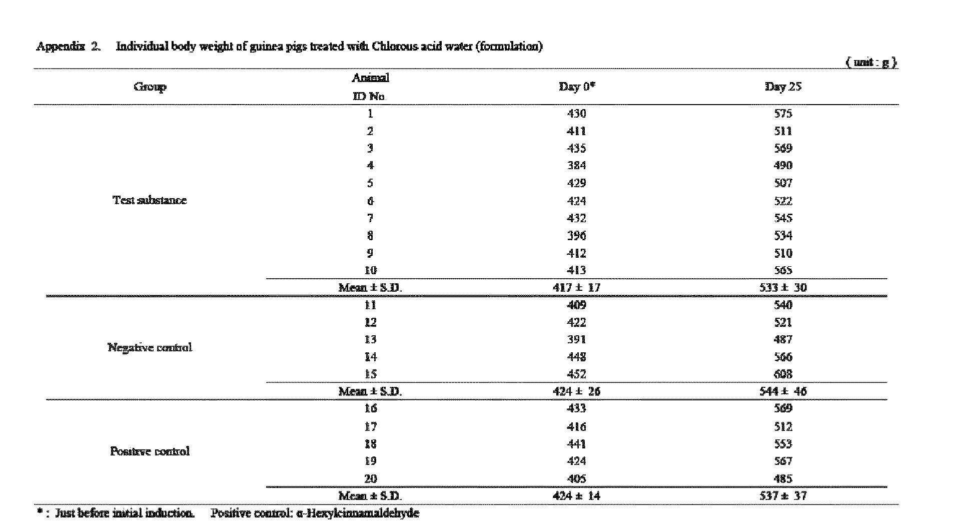

### (Safety of chlorous acid aqueous solution)

In view of the above results, safety of chlorous acid aqueous solutions is shown in comparison to other microbe-removing agents.

### [Chemical Formula 26]

| | Chlorous acid aqueous solution* | Sodium hypochlorite | Ethanol 95 v/v% | Commercially available ethanol 75 v/v% |
|---|---|---|---|---|
| Skin corrosiveness and irritability | not falling under the categories (OECD TG 404) | Category 1 (OECD TG 404) | not falling under the categories (OECD TG 404) | not falling under the categories (OECD TG 404) |
| Severe injury to eye or eye irritability | not falling under the categories (OECD TG 405) | Category 1 (OECD TG 405) | Category 2B (OECD TG 405) | Category 2B (OECD TG 405) |
| Skin sensitization | not falling under the categories (OECD TG 406) | Unable to be classified (insufficient data) | Unable to be classified (insufficient data) | Unable to be classified (insufficient data) |

| | | | | |
|---|---|---|---|---|
| *As chlorous acid aqueous solution, a product with a content of 8000 ppm chlorous acid (HClO₂ = 68.46) and 200 mg/L free chlorine (as Cl at 35.45) or greater was used | | | | |

A liquid for microbe-removal comprising a chlorous acid aqueous solution is under the "not falling under the categories" with respect to skin corrosiveness and irritability, severe injury to eye or eye irritability, and skin sensitization. Specifically, it is demonstrated that a liquid for microbe-removal comprising a chlorous acid aqueous solution, when applied to the skin, does not have a skin corrosive reaction, does not result in erythema or edema, maintains a normal cornea, iris, and conjunctiva when applied to the eye, and does not result in skin sensitization. In contrast, sodium hypochlorite is "category 1" with respect to skin corrosiveness and irritability, and severe injury to eye or eye irritability, meaning that when applied to the skin, erythema and edema are manifested albeit very mildly, and an action that is not expected to be reversible is found on the cornea, iris, or conjunctiva, and/or a positive reaction of a certain score is obtained in corneal turbidity or iritis. Ethanol is "category 2B" or "category 2" with respect to sever injury to eye or eye irritability, meaning that a position reaction of a certain score or higher is obtained in corneal turbidity, iritis, redness of conjunctiva, and/or conjunctival edema. It is understood that a liquid for microbe-removal comprising a chlorous acid aqueous solution, unlike conventional liquid for microbe-removals, is capable of directly removing microbes from animals without inducing an adverse event even when directly contacted with an animal.

A liquid for microbe-removal comprising a chlorous acid aqueous solution can be directly contacted with an animal while having a high microbe-removing performance. Thus, it is demonstrated that such a liquid for microbe-removal is capable of thorough microbe removal by using a disinfection method with a high microbe-removing effect such as a rubbing method, scrubbing method, or a basin method.

As disclosed above, the present disclosure is exemplified by the use of its preferred embodiments. However, it is understood that the scope of the present invention should be interpreted based solely on the Claims. It is also understood that any patent, any patent application, and any other references cited herein should be incorporated herein by reference in the same manner as the contents are specifically described herein.

### [Industrial Applicability]

The liquid for microbe-removal of the present disclosure can be used in removing microbes of animals.

## Claims

1. A liquid for microbe-removal comprising a chlorous acid aqueous solution for removing microbes of an animal by directly contacting the liquid for microbe-removal with the animal.

2. The liquid for microbe-removal of claim 1, wherein the contact is achieved by a rubbing method, a scrubbing method, or a basin method.

3. The liquid for microbe-removal of claim 2, wherein the phosphate buffer comprises two or more types of phosphates.

4. The liquid for microbe-removal of claim 2 or 3, wherein the phosphate buffer comprises two or more of potassium dihydrogen phosphate, dipotassium hydrogen phosphate, and sodium dihydrogen phosphate.

5. The liquid for microbe-removal of claim 4, wherein potassium dihydrogen phosphate is 0.68% to 13.61%, and sodium dihydrogen phosphate is 0.60% to 12.00%.

6. The liquid for microbe-removal of claim 4, wherein potassium dihydrogen phosphate is 1%, and sodium dihydrogen phosphate is 1%.

7. The liquid for microbe-removal of claim 4, wherein potassium dihydrogen phosphate is 0.68% to 13.61%, and dipotassium hydrogen phosphate is 0.87% to 17.42%.

8. The liquid for microbe-removal of claim 4, wherein potassium dihydrogen phosphate is 1%, and dipotassium hydrogen phosphate is 1%.

9. The liquid for microbe-removal of any one of claims 1 to 8, wherein the animal is a live animal.

10. The liquid for microbe-removal of any one of claims 1 to 9, wherein the animal comprises a surface of the animal.

11. The liquid for microbe-removal of claim 10, wherein the surface of the animal is skin.

12. The liquid for microbe-removal of claim 11, wherein the surface of the animal is a hand or a finger.

13. The liquid for microbe-removal of any one of claims 1 to 12, wherein the animal comprises a human body.

14. The liquid for microbe-removal of any one of claims 1 to 13, wherein the removal of microbes is performed during preparation of food.

15. The liquid for microbe-removal of any one of claims 1 to 14, wherein chlorous acid is used at 10 ppm to 60,000 ppm.

16. The liquid for microbe-removal of any one of claims 1 to 15, wherein chlorous acid is used at 200 ppm to 8,000 ppm.

17. The liquid for microbe-removal of any one of claims 1 to 16, wherein a free chlorine concentration of a chlorous acid aqueous solution is 0.25 mg/L to 1,500 mg/L.

18. The liquid for microbe-removal of any one of claims 1 to 17, wherein the removal of microbes targets at least one selected from a virus, a microbe, and a eukaryote.

19. The liquid for microbe-removal of any one of claims 1 to 18, wherein the liquid for microbe-removal does not contain an animal derived component, an alcohol component, or a flavor.

20. A halal-compliant hand and finger liquid for microbe-removal, comprising 1.00% chlorous acid aqueous solution, 1.00% sodium dihydrogen phosphate, 1.00% potassium dihydrogen phosphate, and 97.00% ion exchange water, wherein chlorous acid is 400 ppm, and a free chlorine concentration is 10 mg/L.

21. A halal-compliant hand and finger liquid for microbe-removal, comprising 20.00% chlorous acid aqueous solution, 1.00% potassium dihydrogen phosphate, 1.00% dipotassium hydrogen phosphate, and 78.00% ion exchange water, wherein chlorous acid is 8,000 ppm, and a free chlorine concentration is 200 mg/L.
